# EUROPEAN PATENT APPLICATION

(11) **EP 3 421 483 A1**
(43) Date of publication of application: **02.01.2019**
(21) Application number: 17178707.0
(22) Date of filing: 29.06.2017
(51) Int. Cl.: C07J 43/00, A61K 31/58, A61P 5/32

(54) **NOVEL STEROIDAL 17-BETA HETEROARYL COMPOUNDS AS INHIBITORS OF AKR1C3**

(71) Applicant: Bayer Pharma Aktiengesellschaft, 13353 Berlin (DE)
(72) Inventor: Bothe, Ulrich, 13187 Berlin (DE); Barak, Naomi, 13353 Berlin (DE); Hartung, Ingo, 13158 Berlin (DE); Busemann, Matthias, 13467 Berlin (DE); Fischer, Oliver-Martin, 13127 Berlin (DE)
(74) Representative: BIP Patents

(57) **Abstract**

The invention relates to novel steroidal 17-beta heteroaryl compounds as AKR1C3 inhibitors, to the use thereof for the treatment and/or prophylaxis of diseases and to the use thereof for the production of medicaments for treatment and/or prophylaxis of diseases, especially of gynecological disorders, hyperproliferative disorders, metabolic disorders, or inflammatory disorders as well as prostate cancer including castration-resistant prostate cancer as a sole agent or in combination with other active ingredients.

## Description

### FIELD OF THE INVENTION

The invention relates to AKR1C3 inhibitors, to the use thereof for treatment and/or prophylaxis of diseases and to the use thereof for production of medicaments for treatment and/or prophylaxis of diseases, such as but not limited to gynecological disorders, metabolic disorders and/or inflammatory disorders as well as prostate cancer including castration-resistant prostate cancer.

### BACKGROUND

There is still an unmet medical need for the treatment of gynecological disorders, metabolic disorders and/or inflammatory disorders as well as prostate cancer including castration-resistant prostate cancer.

Endometriosis is a chronic, mainly estrogen-dependent inflammatory disease characterized by the presence of endometrial tissue outside the uterine cavity. Major symptoms of endometriosis are chronic pelvic pain and subfertility.

Estrogen (E2) deprivation is the clinically proven concept and the underlying primary mechanism of action for pharmacological treatment of endometriosis. Besides systemic estrogen levels, there is increasing evidence that locally derived estrogen contributes to the growth of endometriotic lesions. High intra-tissue estrogen concentrations in endometriotic lesions have recently been described, suggesting high local estrogen synthesis in endometriosis (Huhtinen et al. (2012), J Clin Endocrinol Metab.; 97(11):4228-4235). Accordingly, inhibition of local E2 production in the endometriotic lesion is regarded as a highly attractive mechanism of action for the treatment of endometriosis, PCOS and inflammation.

PCOS is a common endocrine disorder, affecting up to 10% of women of reproductive age. It is associated clinically with anovulatory infertility, dysfunctional bleeding, androgen excess, hyperinsulinemia and insulin resistance, obesity and metabolic syndrome (Dunaif et al., (1997) Endocrine Rev. 18; 774-800). Four cardinal features of PCOS have been recognized by the Androgen Excess Society: ovulatory and menstrual dysfunction, biochemical hyperandrogenaemia, clinical hyperandrogonism (e.g. acne, hirsutism) and polycystic ovaries (Azziz et al., (2006) Clin Endocrinol Metab 91 4237-45). The vast majority of women with PCOS will present with clinical signs of hyperandrogonism, e.g. acne, hirsutism, or anovulation manifest by primary subfertility or oligomenorrhea (Legro et al., (2014) N Engl J Med 371: 119-129. Women with PCOS are predisposed to glucose intolerance and metabolic syndrome (Taponen et al., (2004) J of Clin Endocrinology and Metabolism 89: 2114-2118), with associated risk factors for cardiovascular disease and a likely increased risk in future cardiovascular events (Mani et al., (2013) Clin Endocrinol 78: 926-934).

Hyperandrogonism, hirsutism and/or hyperandrogenaemia is the key component of the syndrome and is mandatory for the diagnosis of PCOS (Azziz et al., (2006) Clin Endocrinol Metab 91 4237-45). While serum testosterone is a key factor for biochemical assessment of hyperandrogenaemia, recently androstenedione was suggested as a more reliable marker of PCOS-related androgen excess, since androstenedione is circulating at high concentrations in women with PCOS (O'Reilly et al., (2014) J Clin Endocrinol Metab 99,3: 1027-1036).

PCOS has traditionally been regarded as a disorder of the ovary (Franks et al., (1999) J Steroid Biochem Molecular Biology 69: 269-272). However, increased focus on extra-ovarian and extra-adrenal androgen formation in PCOS has highlighted the role of peripheral tissues such as adipose androgen formation (Quinkler et al., (2004) J of Endocrinology 183. 331-342).

The Aldo-keto reductase family 1 member C3 (AKR1C3 also called type 5 17-beta-hydroxysteroid dehydrogenase (17-beta-HSD5)) is a member of the aldo-keto reductase (AKR) superfamily of enzymes, which reduce the aldehyde/keto group in steroid hormones to the corresponding alcohol and therefore play an important role in androgen-, progesterone-, and estrogene metabolism/activation/deactivation.

AKR1C3 possesses 3α-HSD (hydroxysteroid dehydrogenase activity), 17β-HSD, 20α-HSD and prostaglandin (PG) F synthase activities. It catalyses the conversion of estrone (weak estrogenic activity) to estradiol (potent estrogenic activity), the conversion of progesterone (potent anti-estrogenic activity) to 20-alpha-hydroxyprogesterone (weak anti-estrogenic activity) and the conversion of androstenedione to testosterone (Labrie et al. (2001). Front Neuroendocrinol.; 22(3):185-212). Furthermore AKR1C3 catalyses the conversion of PGH2 to PGF2α and PGD2 to 11β-PGF2, both known to stimulate inflammation and proliferation. Furthermore AKR1C3 has also been shown to metabolize a broad spectrum of carbonyl compounds and xenobiotics, including clinically administered antracyclines (Bains et al. (2010). J. Pharmacol Exp. Ther.; 335: 533-545; Novona et al. (2008). Toxicol Lett.; 181, 1-6. Hofman et al. (2014). Toxicology and Applied Pharmacology 278: 238-248).

AKR1C3 plays a role in several pathologic conditions/diseases such as endometriosis. AKR1C3 is strongly expressed in endometriotic lesions and only marginally detectable in the ovary (Smuc et al. (2009). Mol Cell Endocrinol.; 301(1-2):59-64). In a concerted action with CYP19A1 (aromatase), AKR1C3 is expected to be a key enzyme in local E2 production in endometriotic lesions, generating a pro-estrogenic environment, thereby stimulating proliferation in estrogen-sensitive endometriotic cells. Inhibition of AKR1C3 should therefore result in decreased local intra-tissue E2 levels and thereby decreased proliferation of endometriotic lesions. Effects on ovarian estrogen production are not expected, since AKR1C3 is only marginally expressed in the ovary and 17βHSD1 is the dominant ovarian hydroxysteroid dehydrogenase.

AKR1C3 is also a PGF2α synthase and beside the upregulation of AKR1C3 in endometriotic lesions, it has been shown that levels of PGF2α were significantly higher in both the eutopic and ectopic endometria derived from women with peritoneal endometriosis than in similar tissues derived from women with ovarian endometrioma (Sinreih et al., (2015) Chemico-Biological Interactions; 234: 320-331). PGF2α in endometriotic tissues is expected to contribute to inflammation, pain and proliferation in endometriosis patients and AKR1C3, expressed in endometriotic lesions, is expected to contribute to high local PGF2α level in endometriotic tissues.

AKR1C3 inhibition has the potential to relieve proliferation, pain and inflammation in endometriosis patients by locally reducing E2, testosterone and PGF2α levels in the endometriotic tissues.

AKR1C3 is an androgen-activating enzyme, known to predominantly convert androstenedione to testosterone. Upregulation of AKR1C3 in adipose tissue of PCOS patients has been described, indicating that ARK1C3 expression in adipose is significantly contributing to androgen formation for androstenedione in PCOS patients. It has in addition been shown that AKR1C3 expression in adipocytes is significantly increased by insulin, indicating that insulin, which is high in PCOS is able to drive adipose androgen formation by increasing AKR1C3 activity in female subcutaneous adipose tissue (O'Reilly et., al (2015) Lancet 385 Suppl 1:S16).

AKR1C3 is also a PGF2α synthase and plays a suppressive role in the formation of endogenous ligands for the peroxisome proliferator-activated receptor γ (PPAR-γ receptor), which is a target for insulin-sensitizing drugs (Spiegelman (1998) Diabetes 47:507-514).

Selective AKR1C3 inhibition might offer a novel therapeutic target to reduce androgen burden and improve metabolic phenotype in PCOS. (O'Reilly M1, et al. Lancet. 2015 385 Suppl 1:S16. doi: 10.1016/S0140-6736(15)60331-2; Du et al., (2009) J Clin Endocrinol Metab. 94(7): 2594-2601.) AKR1C3 is involved in prostaglandin biosynthesis, catalyzing the conversion of PGH2 to PGF2α and PGD2 to 11β-PGF2. It has been postulated that expression and upregulation of AKR1C3 supports inflammation by directly causing an increase in 9α,11β-PGF2 synthesis rates and diverting the spontaneous generation of the potent anti-inflammatory mediator 15d-PGJ2 (Mantel et al., (2012) J Invest Dermatol; 132(4): 1103-1110). This function of AKR1C3 has also been implicated in HL-60 cells (Desmond JC et al., (2003) Cancer Res 63: 505-512) and in MCF-7 cells (Byrns MC et al., (2010) J Steroid Biochem Mol Biol 118: 177-187). Inhibition of AKR1C3 is postulated to increase 15d-PGJ2, an anti-inflammatory lipid that mostly mediates its actions directly via activation of PPAR-γ and/or inhibition of NF-κB signaling in immune cells (Maggi et al., (2000) Diabetes 49: 346-355). PGJ2 is described as an anti-inflammatory prostaglandin (Scher JU et al., (2005) Clinical Immunology 114: 100-109). Previous data have shown that PPAR-γ activation attenuates allergen-induced inflammation in skin and lungs of mice (Ward et al., 2006; Dahten et al., 2008). This suggests a role for AKR1C3 inhibition in suppressing of inflammation.

Furthermore AKR1C3 inhibitors have potential for the treatment of prostate hyperplasia (R. O. Roberts et al., Prostate 66(4), 392-404), hair loss (L. Colombe et al., Exp Dermatol 2007, 16(9), 762-769), adiposity (P. A. Svensson et al., Cell Mol Biol Lett 2008, 13(4), 599-613), premature sexual maturity (C. He, Hum Genet 2010, 128(5), 515-527), prostate cancer (Stanbrough M, et al. Increased expression of genes converting adrenal androgens to testosterone in androgen-independent prostate cancer. Cancer Res 2006;66:2815-25; Wako K, et al. Expression of androgen receptor through androgen-converting enzymes is associated with biological aggressiveness in prostate cancer, chronic obstructive pulmonary disease (S. Pierrou, Am J Respir Crit Care 2007, 175(6), 577-586), castration-resistant prostate cancer (Liu C et al. Cancer Res. 2015 Apr 1;75(7):1413-22). (K. M. Fung et al., Endocr Relat Cancer 13(1), 169-180) and antracycline resistant cancer (Fujiwara, A.; Hoshino, T.; Westley, J. W. (1985). "Anthracycline Antibiotics". Critical Reviews in Biotechnology 3 (2):133), Atopic dermatitis (Barr et al., (1988) Br J Pharmacol. 1988; 94:773-80; Satoh et al., (2006) J Immunol.; 177:2621-9.; Shimura et al., (2010) Am J Pathol. 2010; 176:227-37).

Some inhibitors of AKR1C3 are known (review article: Joanna M. Day, Helena J. Tutill, Atul Purohit and Michael J. Reed, Endocrine-Related Cancer (2008) 15, 665-692; see for example also patent applications US20100190826, WO2007/100066, US2014371261, JP2015020966, US20140107085 and P. Bro 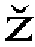 i 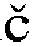 et al., *J. Med. Chem.* **2012,** *55,* 7417-7424, A. O. Adenijii et al., J. Med. Chem. 2012, 55, 2311-2323 and S. M. F. Jamieson et al., J. Med. Chem. 2012, 55, 7746-7758, M. Gazvoda et al., Eur. J. Med. Chem, 2013, 62, 89 - 97; D. M. Heinrich et al., Eur.J. Med. Chem, 2013, 62, 738 - 744; J. U. Flanagan et al., Bioorganic & Medicinal Chemistry, 2014, 22, 967-977; A. Adeniji et al, J. Med. Chem. 2016, 59, 7431-7444, Y. Amano et al., Acta Cryst., 2015, D71, 918-927). An example of a steroidal substance which has been described is EM-1404, based on the oestratriene skeleton with a spirolactone unit at position 17 (F. Labrie et al. US Patent 6,541,463; 2003).

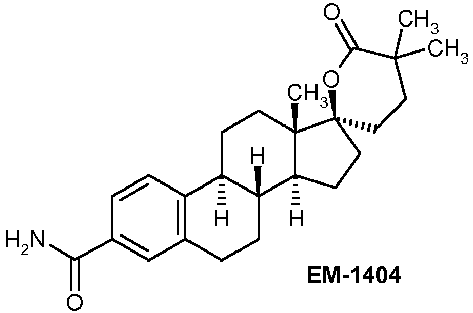

Further steroidal AKR1C3 inhibitors with a lactone unit were described in P. Bydal, Van Luu-The, F. Labrie, D. Poirier, European Journal of Medicinal Chemistry 2009, 44, 632-644. Fluorinated oestratriene derivatives were described in D. Deluca, G. Moller, A. Rosinus, W. Elger, A. Hillisch, J. Adamski, Mol. Cell. Endocrinol. 2006, 248, 218 - 224. Pyrazole and imidazole-dehydroepiandrosterone derivatives with inhibitory activity on AKR1C3 have been reported: M. Cabeza, A. Posada, A. Sanchez-Marquez, Y. Heuze, I. Moreno, J. Soriano, M. Garrido, F. Cortes, E. Bratoeff, J. Enzyme Inhib Med Chem, 2016; 31(1): 53-62, DOI:10.3109/14756366.2014.1003926.

US Patent US 5,604,213 (S. E. Barrie et al.) described 17-(3-pyridyl)oestra-1,3,5(10),16-tetraen-3-ol, a structure substituted on carbon atom 3 by a free hydroxyl group, as a 17a-hydroxylase/C17-20 lyase (Cyp17A1) inhibitor, but not as an AKR1C3 inhibitor.

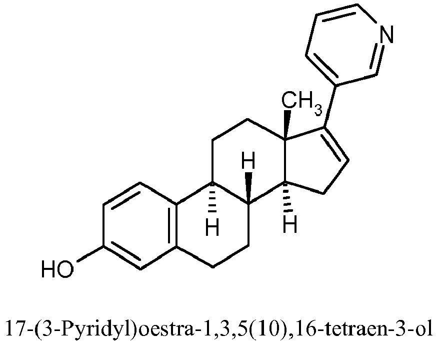

17-(3-Pyridyl)oestra-1,3,5(10),16-tetraene derivatives substituted at position 3 by a carboxamide group are not described in US Patent US 5,604,213. 17β-Heteroaryl substituted compounds based on an estra-1,3,5(10)-triene moiety have not been reported in literature to be active as AKR1C3 inhibitor:
17-(3-Pyridyl)oestra-1,3,5(10),16-tetraene derivates such as compound 13 have been described in WO2014/009274. However, no compound with a carboxamide unit but only compounds with an oxygen atom at the 3-position of the steroidal scaffold have been reported. In addition, all compounds in WO2014009274 display a double bond between position 16 and 17 of the oestrane scaffold.

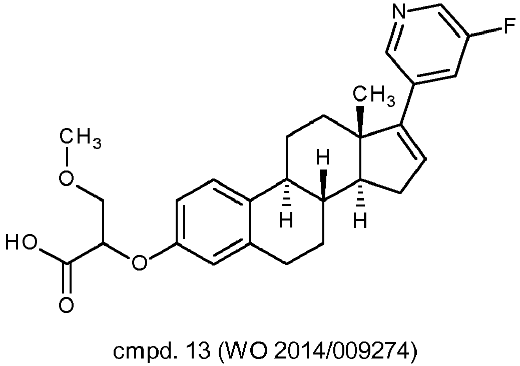

Additional 17-(3-Pyridyl)oestra-1,3,5(10),16-tetraene such as compound 32 have been reported in WO2014128108. However, no compounds displaying a carboxylic acid group linked to the 3-position of the steroid have been described. Moreover, all compounds in WO2014128108 display a double bond between position 16 and 17 of the oestrane scaffold.

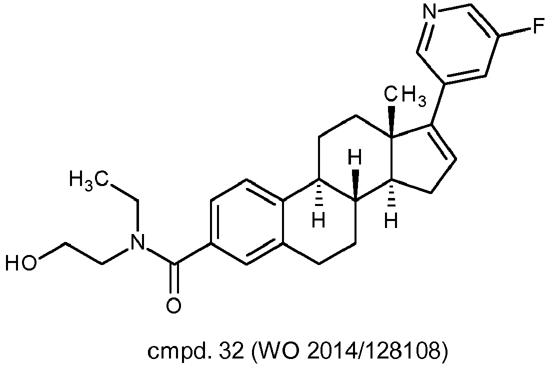

17-(3-Pyridyl)oestra-1,3,5(10),16-tetraene derivates such as compound 2 have been described in WO 2013/045407. All compounds described display a double bond between positon 16 and 17 of the steroidal skeleton.

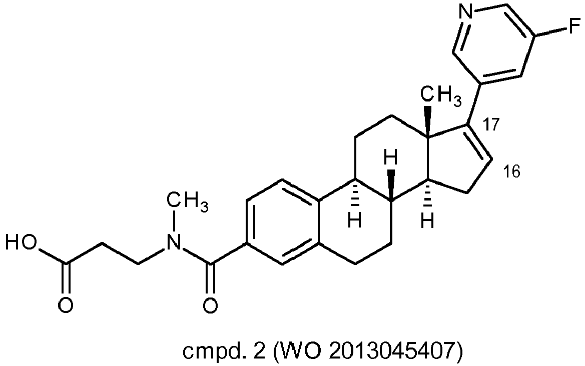

A review of 17-pyridyl- and 17-pyrimidinylandrostane derivatives which are described as Cyp17A1 inhibitors can be found in V. M. Moreira et al. (Current Medicinal Chemistry, 2008 Vol. 15, No. 9).

Although there are already several AKR1C3 inhibitors available there is still an unmet medical need for new medicaments for the treatment of several diseases.

The underlying problem of the present invention to provide new medicaments for gynecological disorders, metabolic disorders and/or inflammatory disorders as well as prostate cancer including castration-resistant prostate cancer is solved by providing alternative AKR1C3 inhibitors. It has now been found, and this constitutes the basis of the present invention, that the compounds of the present invention displaying a single bond between position 16 and 17 and a 17β-pyridine, 17β-pyrimidine, 17β-pyridazine or 17β-pyrazine moiety surprisingly effectively inhibit AKR1C3 for which data are given in the biological experimental section and may therefore be used for the treatment or prophylaxis of AKR1C3 related disorders such as gynecological disorders particularly endometriosis-related and polycystic ovary syndrome-related gynecological disorders, conditions and diseases, metabolic disorders, hyperproliferative disorders, conditions and diseases, and/or inflammation disorders as well as prostate cancer including castration-resistant prostate cancer.

It is an object of the present invention to provide substances active as AKR1C3 inhibitors.

### SUMMARY OF THE INVENTION

In accordance with a first aspect, the present invention covers compounds of general formula (I) 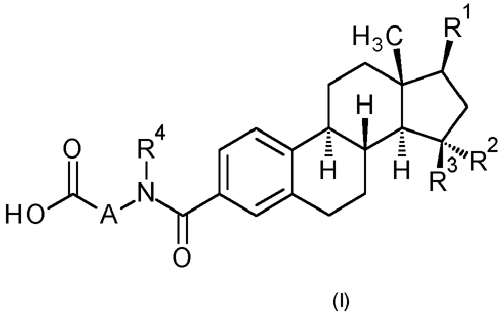 in which
- R¹: repesents a pyridine, pyrimidine, pyridazine or pyrazine moiety which is substituted with R⁵;
- R²: represents hydrogen or hydroxyl;
- R³: represents hydrogen or hydroxyl,
wherein only one of R² and R³ represents hydroxyl;
- R⁴: represents hydrogen or methyl;
- R⁵: represents hydrogen, fluoro, chloro, cyano, C₁-C₄-alkyl, trifluoromethyl or methoxy;
- A: represents -(CR^{a}R^{b})ₙ-
wherein R^{a} and R^{b}, independently from each other, represent hydrogen or methyl;
- n: represents 1, 2, 3 or 4;
and the hydrates, salts, solvates and solvates of the salts thereof.
In a second aspect, the invention relates to compounds of formula (I) as described *supra,* in which
- R¹: represents pyrimidin-5-yl, pyridazin-4-yl, 6-methylpyridazin-4-yl or a group 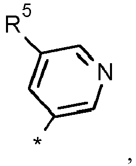 wherein * indicates the point of attachment of said group with the rest of the molecule;
- R²: represents hydrogen or hydroxyl;
- R³: represents hydrogen or hydroxyl;
wherein only one of R² and R³ represents hydroxyl;
- R⁴: represents hydrogen or methyl;
- R⁵: represents hydrogen, fluoro, chloro, methyl, trifluoromethyl or methoxy;
- A: represents -(CR^{a}R^{b})ₙ-
wherein R^{a} and R^{b}, independently from each other, represent hydrogen or methyl;
- n: represents 1, 2, or 3;
and the hydrates, salts, solvates and solvates of the salts thereof.

In a third aspect, the invention relates to compounds of formula (I) as described *supra,* in which
- R¹: represents a group selected from: 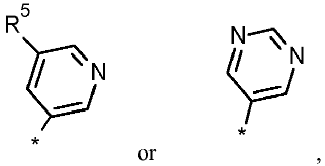 wherein * indicates the point of attachment of said group with the rest of the molecule;
- R²: represents hydrogen or hydroxyl;
- R³: represents hydrogen or hydroxyl;
wherein only one of R² and R³ represents hydroxyl;
- R⁴: represents hydrogen or methyl;
- R⁵: represents fluoro, trifluoromethyl or methoxy;
- A: represents -CH₂-, -CH₂-CH₂-, -CH₂-CH₂-CH₂- or -C(CH₃)₂-CH₂-;
and the hydrates, salts, solvates and solvates of the salts thereof.

In a further aspect of the invention compounds of formula (I) as described above are selected from the group consisting of:
- N-{[(17beta)-17-(5-fluoropyridin-3-yl)estra-1,3,5(10)-trien-3-yl]carbonyl}-N-methyl-beta-alanine
- N-{[(17beta)-17-(5-fluoropyridin-3-yl)estra-1,3,5(10)-trien-3-yl]carbonyl}glycine
- N-{[(17beta)-17-(5-fluoropyridin-3-yl)estra-1,3,5(10)-trien-3-yl]carbonyl}-beta-alanine
- 4-({[(17beta)-17-(5-fluoropyridin-3-yl)estra-1,3,5(10)-trien-3-yl]carbonyl}amino)butanoic acid
- 3-({[(17beta)-17-(5-fluoropyridin-3-yl)estra-1,3,5(10)-trien-3-yl]carbonyl}amino)-2,2-dimethylpropanoic acid
- N-{[(17beta)-17-(5-methoxypyridin-3-yl)estra-1,3,5(10)-trien-3-yl]carbonyl}-N-methyl-beta-alanine
- N-methyl-N-{[(17beta)-17-[5-(trifluoromethyl)pyridin-3-yl]estra-1,3,5(10)-trien-3-yl]carbonyl}-beta-alanine
- N-methyl-N-{[(17beta)-17-[5-(trifluoromethyl)pyridin-3-yl]estra-1,3,5(10)-trien-3-yl]carbonyl}glycine
- N-{[(17beta)-17-[5-(trifluoromethyl)pyridin-3-yl]estra-1,3,5(10)-trien-3-yl]carbonyl}-beta-alanine
- N-methyl-N-{[(17beta)-17-(pyrimidin-5-yl)estra-1,3,5(10)-trien-3-yl]carbonyl}-beta-alanine
- N-{[(15beta,17beta)-17-(5-fluoropyridin-3-yl)-15-hydroxyestra-1,3,5(10)-trien-3-yl]carbonyl}-N-methyl-beta-alanine
- N-{[(15alpha,17beta)-17-(5-fluoropyridin-3-yl)-15-hydroxyestra-1,3,5(10)-trien-3-yl]carbonyl}-N-methyl-beta-alanine
and the hydrates, salts, solvates and solvates of the salts thereof.

Preferred salts in the context of the present invention are physiologically acceptable salts of the inventive compounds. Also encompassed are salts which are not themselves suitable for pharmaceutical applications but can be used, for example, for isolation or purification of the inventive compounds.

Physiologically acceptable salts of the inventive compounds include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalenedisulphonic acid, formic acid, acetic acid, trifluoroacetic acid, propionic acid, lactic acid, tartaric acid, malic acid, citric acid, fumaric acid, maleic acid and benzoic acid.

Physiologically acceptable salts of the inventive compounds also include salts of conventional bases, by way of example and with preference alkali metal salts (e.g. sodium and potassium salts), alkaline earth metal salts (e.g. calcium and magnesium salts) and ammonium salts derived from ammonia or organic amines having 1 to 16 carbon atoms, by way of example and with preference ethylamine, diethylamine, triethylamine, ethyldiisopropylamine, monoethanolamine, diethanolamine, triethanolamine, dicyclohexylamine, dimethylaminoethanol, procaine, dibenzylamine, N-methylmorpholine, arginine, lysine, ethylenediamine and N-methylpiperidine.

In the context of the invention, solvates refer to those forms of the inventive compounds which, in the solid or liquid state, form a complex by coordination with solvent molecules. Hydrates are a specific form of the solvates in which the coordination is with water. Preferred solvates in the context of the present invention are hydrates.

Where the inventive compounds can occur in tautomeric forms, the present invention encompasses all the tautomeric forms.

### DETAILED DESCRIPTION OF THE INVENTION

### DEFINITIONS

The term "C₁-C₄-alkyl" is to be understood as preferably meaning a linear or branched, saturated, monovalent hydrocarbon group having 1, 2, 3 or 4 carbon atoms, e.g. a methyl, ethyl, propyl, butyl, iso-propyl, iso-butyl, sec-butyl, tert-butyl group, more particularly 1, 2 or 3 carbon atoms ("C1-C3-alkyl"), e.g. a methyl, ethyl, n-propyl or iso-propyl group.

The present invention also encompasses all suitable isotopic variants of the inventive compounds. An isotopic variant of an inventive compound is understood here to mean a compound in which at least one atom within the inventive compound has been exchanged for another atom of the same atomic number, but with a different atomic mass from the atomic mass which usually or predominantly occurs in nature. Examples of isotopes which can be incorporated into an inventive compound are those of hydrogen, carbon, nitrogen, oxygen, phosphorus, sulphur, fluorine, chlorine, bromine and iodine, such as ²H (deuterium), ³H (tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I and ¹³¹I. Particular isotopic variants of an inventive compound, especially those in which one or more radioactive isotopes have been incorporated, may be beneficial, for example, for the examination of the mechanism of action or of the active ingredient distribution in the body; due to comparatively easy preparability and detectability, especially compounds labelled with ³H or ¹⁴C isotopes are suitable for this purpose. In addition, the incorporation of isotopes, for example of deuterium, can lead to particular therapeutic benefits as a consequence of greater metabolic stability of the compound, for example an extension of the half-life in the body or a reduction in the active dose required; such modifications of the inventive compounds may therefore in some cases also constitute a preferred embodiment of the present invention. Isotopic variants of the inventive compounds can be prepared by the processes known to those skilled in the art, for example by the methods described below and the instructions reproduced in the working examples, by using corresponding isotopic modifications of the particular reagents and/or starting compounds therein.

Moreover, the present invention also encompasses prodrugs of the inventive compounds. The term "prodrugs" here denotes compounds which may themselves be biologically active or inactive, but are converted (for example metabolically or hydrolytically) to inventive compounds during their residence time in the body.

In the context of the present invention, the term "treatment" or "treating" includes inhibition, retardation, checking, alleviating, attenuating, restricting, reducing, suppressing, repelling or healing of a disease, a condition, a disorder, an injury or a health problem, or the development, the course or the progression of such states and/or the symptoms of such states. The term "therapy" is understood here to be synonymous with the term "treatment".

The terms "prevention", "prophylaxis" or "preclusion" are used synonymously in the context of the present invention and refer to the avoidance or reduction of the risk of contracting, experiencing, suffering from or having a disease, a condition, a disorder, an injury or a health problem, or a development or advancement of such states and/or the symptoms of such states.

The treatment or prevention of a disease, a condition, a disorder, an injury or a health problem may be partial or complete.

The present invention provides compounds of the formula (I) which have an unforeseeable, valuable spectrum of pharmacological and pharmacokinetic activity. They are therefore suitable for use in the treatment or prophylaxis of a disease. Furthermore compounds of the formula (I) are suitable for use as medicaments for treatment and/or prophylaxis of disorders in humans and animals. The term "treatment" in the context of the present invention includes prophylaxis. The pharmaceutical efficacy of the inventive compounds can be explained by the action thereof as an AKR1C3 inhibitor. As shown in Table 3, the inventive compounds are potent inhibitors of the AKR1C3 enzyme.

The inventive compounds are suitable for the treatment and/or prophylaxis of endometriosis and in addition, for treatment and/or prophylaxis of uterine leiomyoma, of uterine bleeding disorders, of dysmenorrhoea, of prostate cancer including castration-resistant prostate cancer, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity, or of inflammation-related pain.

The present invention further provides for the use of the inventive compounds for production of a medicament for treatment and/or prophylaxis of disorders, especially of the aforementioned disorders.

The present invention further provides a method for treatment and/or prophylaxis of disorders, especially the aforementioned disorders, using an effective amount of the inventive compounds.

The present invention further provides for the use of the inventive compounds for treatment and/or prophylaxis of disorders, especially of the aforementioned disorders.

The present invention further provides the inventive compounds for use in a method for treatment and/or prophylaxis of the aforementioned disorders.

The present invention further provides medicaments comprising at least one inventive compound and at least one or more than one further active ingredient, especially for treatment and/or prophylaxis of the aforementioned disorders. Preferred examples of suitable combination active ingredients include: selective oestrogen receptor modulators (SERMs), oestrogen receptor (ER) antagonists, aromatase inhibitors, 17β-HSD1 inhibitors, steroid sulphatase (STS) inhibitors, GnRH agonists and antagonists, kisspeptin receptor (KISSR) antagonists, selective androgen receptor modulators (SARMs), androgens, 5α-reductase inhibitors, selective progesterone receptor modulators (SPRMs), gestagens, antigestagens, oral contraceptives, inhibitors of mitogen-activated protein (MAP) kinases and inhibitors of the MAP kinases (Mkk3/6, Mek1/2, Erk1/2), inhibitors of the protein kinases B (PKBα/β/γ; Akt1/2/3), inhibitors of the phosphoinositide 3-kinases (PI3K), inhibitors of cyclin-dependent kinase (CDK1/2), inhibitors of the hypoxia-induced signalling pathway (HIF1alpha inhibitors, activators of prolylhydroxylases), histone deacetylase (HDAC) inhibitors, prostaglandin F receptor (FP) (PTGFR) antagonists, and non-steroidal inflammation inhibitors (NSAIDs).

For example, the compounds of the present invention can be combined with known antihyperproliferative, cytostatic or cytotoxic substances for treatment of cancers. In addition, the inventive compounds can also be used in combination with radiotherapy and/or surgical intervention.

Examples of suitable combination active ingredients include:
131I-chTNT, abarelix, abiraterone, aclarubicin, aldesleukin, alemtuzumab, alitretinoin, altretamine, aminoglutethimide, amrubicin, amsacrine, anastrozole, arglabin, arsentrioxidas, asparaginase, azacitidine, basiliximab, RDEA 119, belotecan, bendamustine, bevacizumab, bexarotene, bicalutamide, bisantrene, bleomycin, bortezomib, buserelin, busulfan, cabazitaxel, calcium folinate, calcium levofolinate, capecitabine, carboplatin, carmofur, carmustine, catumaxomab, celecoxib, celmoleukin, cetuximab, chlorambucil, chlormadinone, chlormethine, cisplatin, cladribine, clodronic acid, clofarabine, crisantaspase, cyclophosphamide, cyproterone, cytarabine, dacarbazine, dactinomycin, darbepoetin alfa, dasatinib, daunorubicin, decitabine, degarelix, denileukin diftitox, denosumab, deslorelin, dibrospidium chloride, docetaxel, doxifluridine, doxorubicin, doxorubicin + oestrone, eculizumab, edrecolomab, elliptinium acetate, eltrombopag, endostatin, enocitabine, epirubicin, epitiostanol, epoetin alfa, epoetin beta, eptaplatin, eribulin, erlotinib, oestradiol, oestramustine, etoposide, everolimus, exemestane, fadrozole, filgrastim, fludarabine, fluorouracil, flutamide, formestane, fotemustine, fulvoestrant, gallium nitrate, ganirelix, gefitinib, gemcitabine, gemtuzumab, glutoxim, goserelin, histamine dihydrochloride, histrelin, hydroxycarbamide, I-125 pellets, ibandronic acid, ibritumomab tiuxetan, idarubicin, ifosfamide, imatinib, imiquimod, improsulfan, interferon alfa, interferon beta, interferon gamma, ipilimumab, irinotecan, ixabepilone, lanreotide, lapatinib, lenalidomide, lenograstim, lentinan, letrozole, leuprorelin, levamisole, lisuride, lobaplatin, lomustine, lonidamine, masoprocol, medroxyprogesterone, megestrol, melphalan, mepitiostan, mercaptopurine, methotrexate, methoxsalen, methyl aminolevulinate, methyltestosterone, mifamurtide, miltefosine, miriplatin, mitobronitol, mitoguazone, mitolactol, mitomycin, mitotane, mitoxantrone, nedaplatin, nelarabine, nilotinib, nilutamide, nimotuzumab, nimustine, nitracrine, ofatumumab, omeprazole, oprelvekin, oxaliplatin, p53 gene therapy, paclitaxel, palifermin, palladium-103 pellets, pamidronic acid, panitumumab, pazopanib, pegaspargase, pEG-epoetin beta (methoxy PEG-epoetin beta), pegfilgrastim, peginterferon alfa-2b, pemetrexed, pentazocine, pentostatin, peplomycin, perfosfamid, picibanil, pirarubicin, plerixafor, plicamycin, poliglusam, polyoestradiol phosphate, polysaccharide-K, porfimer sodium, pralatrexate, prednimustine, procarbazine, quinagolide, radium-223 chloride, raloxifen, raltitrexed, ranimustine, razoxane, regorafenib, risedronic acid, rituximab, romidepsin, romiplostim, sargramostim, sipuleucel-T, sizofiran, sobuzoxan, sodium glycididazole, sorafenib, streptozocin, sunitinib, talaporfin, tamibarotene, tamoxifen, tasonermin, teceleukin, tegafur, tegafur + gimeracil + oteracil, temoporfin, temozolomide, temsirolimus, teniposide, testosterone, tetrofosmin, thalidomide, thiotepa, thymalfasin, tioguanine, tocilizumab, topotecan, toremifene, tositumomab, trabectedin, trastuzumab, treosulfan, tretinoin, trilostane, triptorelin, trofosfamide, tryptophan, ubenimex, valrubicin, vandetanib, vapreotide, vemurafenib, vinblastine, vincristine, vindesine, vinflunine, vinorelbine, vorinostat, vorozole, yttrium-90 glass microspheres, zinostatin, zinostatin stimalamer, zoledronic acid, zorubicin.

The present invention preferably relates to medicaments comprising at least one inventive compound and one or more of the following active ingredients, especially for treatment and/or prophylaxis of androgen receptor-dependent proliferative disorders:
- LHRH (luteinizing hormone-releasing hormone) agonists,
- LHRH (luteinizing hormone-releasing hormone) antagonists,
- C(17,20)-lyase inhibitors,
- type I 5-α-reductase inhibitors,
- type II 5-α-reductase inhibitors,
- mixed type I/II 5-α-reductase inhibitors,
- α-radiation-emitting radiopharmaceuticals for treatment of bone metastases, for example radium-223 chloride,
- cytostatics,
- VEGF (Vascular Endothelial Growth Factor) kinase inhibitors,
- antigestagens,
- antioestrogens,
- EGF antibodies,
- oestrogens or
- other androgen receptor antagonists,
- poly(ADP-ribose) polymerase I inhibitors, or
- bi-specific T-cell engagers (BiTE) coupled to a cell surface protein, for example prostate-specific membrane antigen (PSMA).

The invention also relates to pharmaceutical composition comprising a compound of the general formula I (or physiologically acceptable addition salts with organic and inorganic acids) and to the use of these compounds for production of medicaments, especially for the aforementioned indications. The pharmaceutical composition comprising a compound of general formula (I) can also contain one or more pharmaceutically acceptable excipients. The pharmaceutical combination can also contain one or more first active ingredients, in particular compounds of general formula (I) and one or more further active ingredients.

The compounds can be used for the aforementioned indications after either oral or parenteral administration.

A pharmaceutical combination can contain one or more further active ingredients which is selected from the group of anti androgens, CYP17A1 inhibitors, 5 alpha reductase inhibitors, GNRHa and GNRH antagoists, or LHRH agonists for example Flutamide, Bicalutamide, Nilutamide, Enzaluatmide, ODM-201, abiraterone and abiraterone metabolites, finasteride dutasteride, Leuprolide, Goserelin, Triptorelin, Histrelin, or Degarelix.

The inventive compounds can act systemically and/or locally. For this purpose, they can be administered in a suitable manner, for example by the oral, parenteral, pulmonal, nasal, sublingual, lingual, buccal, rectal, dermal, transdermal, conjunctival, otic route, or as an implant or stent.

The inventive compounds can be administered in administration forms suitable for these administration routes.

Suitable administration forms for oral administration are those which release the inventive compounds in a rapid and/or modified manner, work according to the prior art and contain the inventive compounds in crystalline and/or amorphous and/or dissolved form, for example tablets (uncoated or coated tablets, for example with enteric or retarded-dissolution or insoluble coatings which control the release of the inventive compound), tablets or films/wafers which disintegrate rapidly in the oral cavity, films/lyophilizates, capsules (for example hard or soft gelatin capsules), sugar-coated tablets, granules, pellets, powders, emulsions, suspensions, aerosols or solutions.

Parenteral administration can be accomplished with avoidance of an absorption step (for example by an intravenous, intraarterial, intracardial, intraspinal or intralumbar route) or with inclusion of an absorption (for example by an intramuscular, subcutaneous, intracutaneous, percutaneous or intraperitoneal route). Suitable administration forms for parenteral administration include injection and infusion formulations in the form of solutions, suspensions, emulsions, lyophilizates or sterile powders.

For the other administration routes, suitable examples are inhalation medicaments (including powder inhalers, nebulizers), nasal drops, solutions or sprays; tablets for lingual, sublingual or buccal administration, films/wafers or capsules, suppositories, ear or eye preparations, vaginal capsules, aqueous suspensions (lotions, shaking mixtures), lipophilic suspensions, ointments, creams, transdermal therapeutic systems (for example patches), milk, pastes, foams, dusting powders, implants, intrauterine coils, vaginal rings or stents.

The inventive compounds can be converted to the administration forms mentioned. This can be done in a manner known per se, by mixing with inert, nontoxic, pharmaceutically suitable excipients. These excipients include carriers (for example microcrystalline cellulose, lactose, mannitol), solvents (e.g. liquid polyethylene glycols), emulsifiers and dispersing or wetting agents (for example sodium dodecylsulphate, polyoxysorbitan oleate), binders (for example polyvinylpyrrolidone), synthetic and natural polymers (for example albumin), stabilizers (e.g. antioxidants, for example ascorbic acid), dyes (e.g. inorganic pigments, for example iron oxides) and flavour and/or odour correctants.

The present invention further provides medicaments which comprise at least one inventive compound, typically together with one or more inert, nontoxic, pharmaceutically suitable excipients, and the use thereof for the aforementioned purposes.

In the case of oral administration, the amount is about 0.01 to 100 mg/kg of body weight per day. The amount of a compound of the formula I to be administered varies within a wide range and can cover any effective amount. Depending on the condition to be treated and the mode of administration, the amount of the compound administered may be 0.01 - 100 mg/kg of body weight per day.

In spite of this, it may be necessary to deviate from the amounts specified, specifically depending on body weight, administration route, and individual behaviour towards the active ingredient, type of formulation, and time or interval of administration. For instance, less than the aforementioned minimum amount may be sufficient in some cases, while the upper limit mentioned has to be exceeded in other cases. In the case of administration of greater amounts, it may be advisable to divide them into several individual doses over the day.

The percentages in the tests and examples which follow are, unless indicated otherwise, percentages by weight; parts are parts by weight. Solvent ratios, dilution ratios and concentration data for liquid/liquid solutions are based in each case on volume.

The present invention further provides medicaments for treatment and prophylaxis of endometriosis, of uterine leiomyoma, of uterine bleeding disorders, of dysmenorrhoea, of prostate carcinoma, of prostate hyperplasia, of acne, of seborrhoea, of hair loss, of premature sexual maturity, of polycystic ovary syndrome, of breast cancer, of lung cancer, of endometrial carcinoma, of renal cell carcinoma, of bladder carcinoma, of non-Hodgkins lymphoma, of chronic obstructive pulmonary disease (COPD), of obesity, or of inflammation-related pain.

The present invention further provides for the use of the compounds of the formula (I) in the form of a pharmaceutical formulation for enteral, parenteral, vaginal, intrauterine and oral administration.

**Table 1: List of chemical abbreviations**

| Abbreviations and acronyms: | |
|---|---|
| DMF | *N,N-*dimethylformamide |
| DMSO | dimethyl sulphoxide |
| NMP | 1-methylpyrrolidin-2-one |
| THF | tetrahydrofuran |
| h | hour(s) |
| tBuOH | *tert*-Butyl alcohol |
| HPLC | high-pressure, high-performance liquid chromatography |
| LC-MS | liquid chromatography-coupled mass spectroscopy |
| ES-MS | electrospray mass spectrometry |
| min | minute(s) |
| MS | mass spectrometry |
| NMR | nuclear magnetic resonance spectroscopy |
| Rt | retention time |
| TFA | trifluoroacetic acid |
| room temp. | room temperature |

**Table 2: Structure analysis of the inventive compounds:**

| In the NMR data of the inventive compounds, the following meanings apply: | |
|---|---|
| s | singlet |
| d | doublet |
| t | triplet |
| q | quartet |
| quin | quintet |
| m | multiplet |
| br | broad |
| mc | centred multiplet |

The various aspects of the invention described in this application are illustrated by the following examples which are not meant to limit the invention in any way.

The example testing experiments described herein serve to illustrate the present invention and the invention is not limited to the examples given.

### EXPERIMENTAL SECTION - GENERAL PART

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil^{®} or KP-NH^{®} in combination with a Biotage autopurifier system (SP4^{®} or Isolera Four^{®}) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

### UPLC-MS Standard Procedures

Analytical UPLC-MS was performed as described below. The masses (m/z) are reported from the positive mode electrospray ionisation unless the negative mode is indicated (ESI-). In most of the cases method 1 is used. If not, it is indicated.

### Method A1: UPLC (ACN-HCOOH):

Instrument: Waters Acquity UPLC-MS SQD 3001; column: Acquity UPLC BEH C18 1.7 50x2.1 mm; mobile phase A: water + 0.1% by volume of formic acid (99%), mobile phase B: acetonitrile; gradient: 0-1.6 min 1-99% B, 1.6-2.0 min 99% B; flow rate 0.8 ml/min; temperature: 60°C; injection: 2 µl; DAD scan: 210-400 nm; ELSD.

### Method A2: UPLC (ACN-NH3):

Instrument: Waters Acquity UPLC-MS SQD 3001; column: Acquity UPLC BEH C18 1.7 50x2.1 mm; mobile phase A: water + 0.2% by volume of ammonia (32%), mobile phase B: acetonitrile; gradient: 0-1.6 min 1-99% B, 1.6-2.0 min 99% B; flow rate 0.8 ml/min; temperature: 60°C; injection: 2 µl; DAD scan: 210-400 nm; ELSD.

### Preparative HPLC methods:

Method P1: System: Waters autopurification system: Pump 2545, Sample Manager 2767, CFO, DAD 2996, ELSD 2424, SQD; column: XBridge C18 5µm 100x30 mm; mobile phase: A: water + 0.1% by volume of formic acid, mobile phase B: acetonitrile; gradient: 0-8 min 10-100% B, 8-10 min 100% B; flow rate: 50 ml/min; temperature: room temp.; solution: max. 250 mg / max. 2.5 ml DMSO or DMF; injection: 1 x 2.5 ml; detection: DAD scan range 210-400 nm; MS ESI+, ESI-, scan range 160-1000 m/z.
Method P2: System: Waters autopurification system: Pump 254, Sample Manager 2767, CFO, DAD 2996, ELSD 2424, SQD 3100; column: XBridge C18 5µm 100x30 mm; mobile phase: A: water + 0.2% by volume of ammonia (32%), mobile phase B: methanol; gradient: 0-8 min 30-70% B; flow rate: 50 ml/min; temperature: room temp.; detection: DAD scan range 210-400 nm; MS ESI+, ESI-, scan range 160-1000 m/z; ELSD.

### EXPERIMENTAL SECTION GENERAL PROCEDURES

### Synthesis of the compounds

Three routes for the preparation of compounds of general formula (I) are described in schemes 1, 2 and 3.

### Scheme 1: Synthesis of compounds of general formula (I)-A representing a subset of compounds of general formula (I), in which R1, R4 and A have the meaning as given for general formula (I), supra.

As depicted in scheme 1, compounds of the general formula (II) (which have been described or can be prepared analogously to procedures reported in WO2013045407) can be transformed to compounds of formula (III) using palladium on charcoal under a hydrogen gas atmosphere in a suitable solvent such as ethylacetate, THF or methanol or mixtures thereof. Compounds of formula (III) can then be transformed to compounds of general formula (I)-A representing a subset of compounds of inventive compounds of formula (I) using methods as described in WO2013045407.

In particular, compounds of general formula (I)-A can be obtained by amide formation reactions using compounds of the general formula (III) with suitably protected amino acids and subsequent removal of the protecting group. Preferred is the transformation of compounds of general formula (III) with *tert-*butylesters derived from amino acids using 1-hydroxybenzotriazole hydrate, 1-ethyl-3-(3-dimethylaminopropyl)carbodiimide hydrochloride and triethylamine. Subsequent treatment of the *tert-*butyl protected product with trifluoroacetic acid in dichloromethane leads to compounds of general formula (I)-A. Also preferred for the transformation of compound of general formula (III) to compounds of formula (I)-A is the reaction with methyl or ethylesters derived from amino acids and subsequent deprotection by saponification using aqeous sodium hydroxide solution.

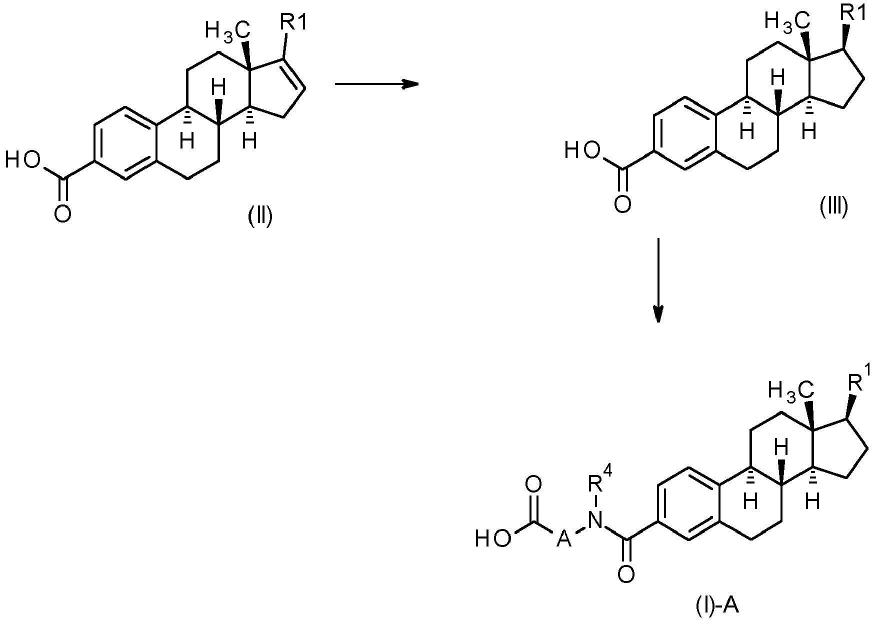

### Scheme 2: Synthesis of compounds of general formula (I)-B, in which R¹, R⁴ and A have the meaning as given for general formula (I), supra.

As depicted in scheme 2, compounds of the general formula (I)-B representing a subset of inventive compounds of formula (I) can be synthesized applying a microbiological oxidation. Preferred is the use of the strain Mucor plumbeus (CBS No. 29563).

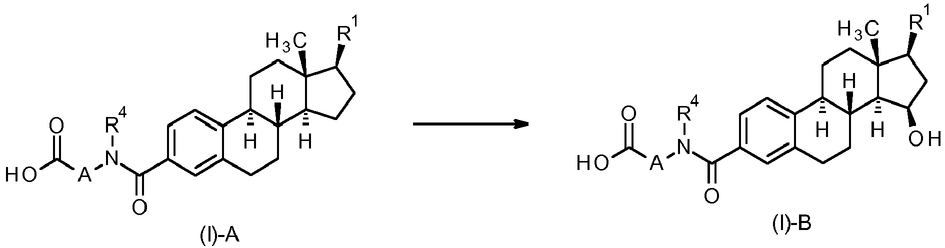

### Scheme 3: Synthesis of compounds of general formula (I)-C, in which R¹, R⁴ and A have the meaning as given for general formula (I), supra.

As depicted in scheme 3, compounds of the general formula (I)-C representing a subset of inventive compounds of formula (I) can be synthesized applying a microbiological oxidation. Preferred is the use of strain Calonectria decora (ATCC No. 14767).

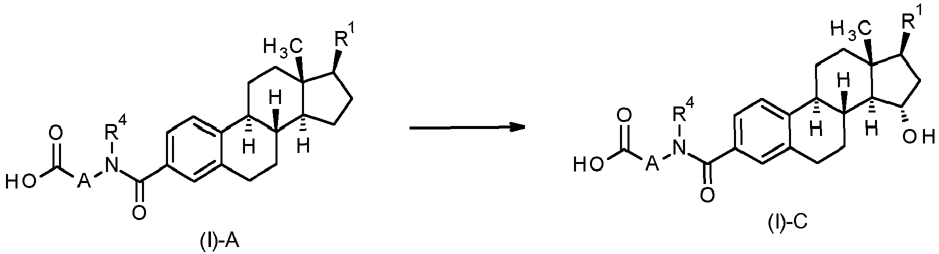

### EXPERIMENTAL SECTION -INTERMEDIATES

### Intermediate 1-1

### (17beta)-17-(5-fluoropyridin-3-yl)estra-1,3,5(10)-triene-3-carboxylic acid

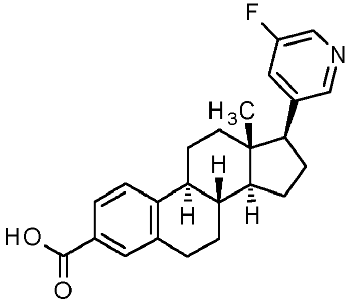

A mixture of 200 mg palladium (Pd) on charcoal (10% Pd), 143 mg 17-(5-fluoropyridin-3-yl)estra-1,3,5(10),16-tetraene-3-carboxylic acid (intermediate 3-a of WO2013045407), 8 ml ethyl acetate and 8 ml THF was stirred under hydrogen atmosphere for 1.5 h. Then 200 mg Pd on charcoal were added and the mixture was stirred under hydrogen atmosphere for 1.5 h. Again 200 mg Pd on charcoal were added and the mixture was stirred under hydrogen atmosphere for 1.5 h. The mixture was filtered and the filter cake was washed with a mixture of ethyl acetate and THF. Evaporation of solvents afforded 100 mg of (17beta)-17-(5-fluoropyridin-3-yl)estra-1,3,5(10)-triene-3-carboxylic acid as a crude product.

1H-NMR (300MHz, DMSO-d6, selected signals): δ [ppm]= 0.44 (s, 3H), 1.69 - 1.76 (m, 1H), 2.78 - 2.91 (m, 3H), 3.52 - 3.60 (m, 1H), 7.35 (d, 1H), 7.57 - 7.67 (m, 3H), 8.30 (s, 1H), 8.38 (d, 1H), 12.66 (br s, 1H).

### Intermediate 1-2

### (17beta)-17-[5-(trifluoromethyl)pyridin-3-yl]estra-1,3,5(10)-triene-3-carboxylic acid

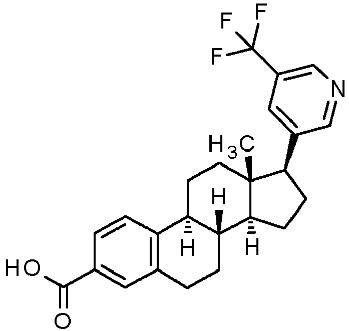

189 mg 17-[5-(trifluoromethyl)pyridin-3-yl]estra-1,3,5(10),16-tetraene-3-carboxylic acid (intermediate 3-d of WO2013045407) was dissolved in 10 ml of a 3:1 mixture of ethyl acetate and THF. 250 mg palladium on charcoal was added and the mixture was stirred for 2 h under a hydrogen atmosphere. The filter cake was washed with a mixture of ethyl acetate and THF and then washed with DMSO. The fitrate was partly evaporated and then treated with water leading to the precipitation of a solid. The solid was filtered and washed with water, dried and purified by HPLC. The resulting product was then stirred in diethylether, filtered and dried leading to 50 mg of (17beta)-17-[5-(trifluoromethyl)pyridin-3-yl]estra-1,3,5(10)-triene-3-carboxylic acid. ¹H-NMR (300MHz, DMSO-d₆): δ [ppm]= 0.43 (s, 3H), 1.19 - 1.67 (m, 7H), 1.77 - 2.01 (m, 3H), 2.13 - 2.42 (m, 3H), 2.80 - 2.96 (m, 3H), 7.34 (d, 1H), 7.59 - 7.66 (m, 2H), 8.00 (s, 1H), 8.74 (br s, 1H), 8.80 (s, 1H), 12.69 (br s, 1H).

### Intermediate 1-3

### (17beta)-17-(5-methoxypyridin-3-yl)estra-1,3,5(10)-triene-3-carboxylic acid

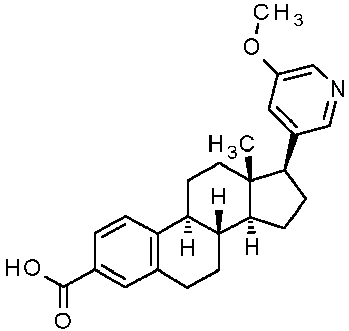

A mixture of 0.80 g 17-(5-methoxypyridin-3-yl)estra-1,3,5(10),16-tetraene-3-carboxylic acid (intermediate 3-b of WO2013045407) in 60 ml ethyl acetate, 5 ml methanol and THF was treated with 1.1 g palladium on charcoal and the mixture was stirred for 2 h at room temperature. The mixture was filtered, the filter cake was washed with ethylacetate and the filtrate was evaporated leading to 511 mg the title compound.

¹H-NMR (300MHz, DMSO-d₆): δ [ppm]= 0.44 (s, 3H), 1.17 - 1.56 (m, 7H), 1.72 - 1.98 (m, 3H), 2.06 - 2.37 (m, 3H), 2.68 - 2.91 (m, 3H), 3.79 (s, 3H), 7.19 (s, 1H), 7.34 (d, 1H), 7.58 - 7.66 (m, 2H), 8.03 (s, 1H), 8.11 (d, 1H), 12.68 (br s, 1H).

### Intermediate 1-4

### (17beta)-17-(pyrimidin-5-yl)estra-1,3,5(10)-triene-3-carboxylic acid

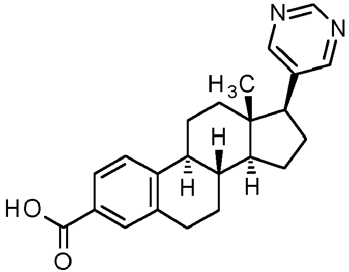

0.90 g 17-(pyrimidin-5-yl)estra-1,3,5(10),16-tetraene-3-carboxylic acid (intermediate 3-c of WO2013045407) were transformed to 223 mg (17beta)-17-(pyrimidin-5-yl)estra-1,3,5(10)-triene-3-carboxylic acid (crude product) similar to the procedure for the synthesis of intermediate 1-3.

¹H-NMR (400MHz, DMSO-d₆, selected data): δ [ppm]= 0.46 (s, 3H), 2.76 (t, 1H), 2.82 - 2.90 (m, 2H), 7.35 (d, 1H), 7.61 (s, 1H), 7.63 (d, 1H), 8.69 (s, 2H), 9.02 (s, 1H), 12.66 (br s, 1H).

### Intermediate 2-1

### tert-butyl N-[[(17beta)-17-(5-fluoropyridin-3-yl)estra-1,3,5(10)-trien-3-yl]carbonyl}-N-methyl-beta-alaninate

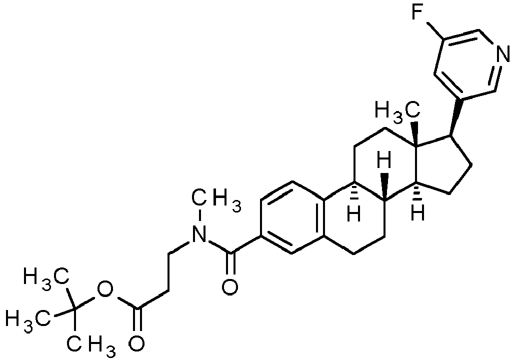

A mixture of 1.2 g (17beta)-17-(5-fluoropyridin-3-yl)estra-1,3,5(10)-triene-3-carboxylic acid (Intermediate 1-1) and 1.007 g tert-butyl N-methyl-beta-alaninate in 60 ml THF and 2.5 ml DMF was treated with 0.48 g 1-hydroxybenzotriazole hydrate, 1.21 g 1-ethyl-3-(3-dimethylaminopropyl)carbodiimide hydrochloride and 1.3 ml triethylamine and was stirred for 3 days. Water was added and the mixture was extracted three times by ethyl acetate. The organic layer was washed with brine and evaporated. The residue was purified by column chromatography on silica (hexane/ethyl acetate) leading to 1.0 g of tert-butyl N-{[(17beta)-17-(5-fluoropyridin-3-yl)estra-1,3,5(10)-trien-3-yl]carbonyl}-N-methyl-beta-alaninate.

¹H-NMR (300MHz, CHLOROFORM-d, selected signals): δ [ppm]= 0.54 (s, 3H), 1.33 - 1.56 (m, contains singulett at 1.45 ppm), 1.38 - 1.54 (m, 14H), 1.58 (s, 2H), 1.69 - 1.79 (m, 1H), 1.88 - 2.17 (m, 4H), 2.26 - 2.70 (4H), 2.78 - 2.97 (m, 3H), 3.02 (br s, 3H), 3.5 - 3.84 (2H), 7.08 - 7.18 (m, 2H), 7.30 (d, 1H), 8.29 - 8.35 (m, 2H).

### Intermediate 2-2

### tert-butyl N-methyl-N-{[(17beta)-17-[5-(trifluoromethyl)pyridin-3-yl]estra-1,3,5(10)-trien-3-yl] carbonyl}-beta-alaninate

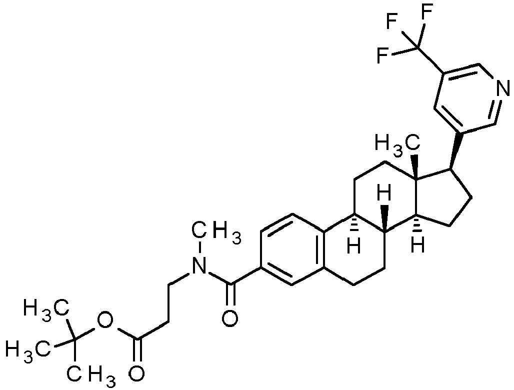

A mixture of 100 mg (17beta)-17-[5-(trifluoromethyl)pyridin-3-yl]estra-1,3,5(10)-triene-3-carboxylic acid (Intermediate 1-2) and 74 mg tert-butyl N-methyl-beta-alaninate in 3 ml THF and 0.5 ml DMF was treated with 36 mg 1-hydroxybenzotriazole hydrate, 89 mg 1-ethyl-3-(3-dimethylaminopropyl)carbodiimide hydrochloride and 0.1 ml triethylamine and was stirred for 18 h. Water was added and the mixture was extracted three times by ethyl acetate. The organic layer was evaporated leading to 141 mg tert-butyl N-methyl-N-{[(17beta)-17-[5-(trifluoromethyl)pyridin-3-yl]estra-1,3,5(10)-trien-3-yl]carbonyl}-beta-alaninate (crude product).

### Intermediate 2-3

### tert-butyl N-{[(17beta)-17-(5-methoxypyridin-3-yl)estra-1,3,5(10)-trien-3-yl]carbonyl}-N-methyl-beta-alaninate

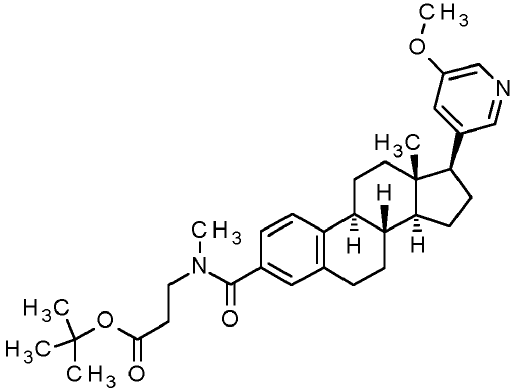

A mixture of 100 mg (17beta)-17-(5-methoxypyridin-3-yl)estra-1,3,5(10)-triene-3-carboxylic acid (intermediate 1-3) and 81 mg tert-butyl N-methyl-beta-alaninate in 3 ml THF and 0.5 ml DMF was treated with 39 mg 1-hydroxybenzotriazole hydrate, 100 mg 1-ethyl-3-(3-dimethylaminopropyl)carbodiimide hydrochloride and 0.11 ml triethylamine and was stirred for 18 h. Water was added and the mixture was extracted three times by ethyl acetate. The organic layer was evaporated leading to 174 mg tert-butyl N-{[(17beta)-17-(5-methoxypyridin-3-yl)estra-1,3,5(10)-trien-3-yl]carbonyl}-N-methyl-beta-alaninate (crude product).

### Intermediate 2-4

### tert-butyl N-methyl-N-{[(17beta)-17-(pyrimidin-5-yl)estra-1,3,5(10)-trien-3-yl]carbonyl}-beta-alaninate

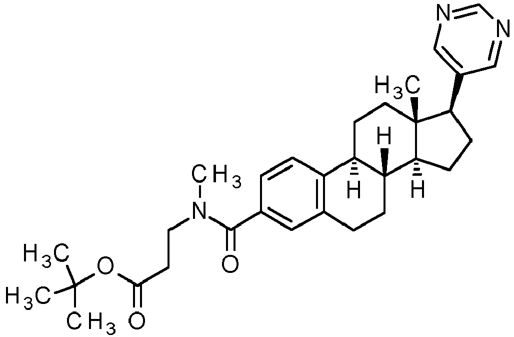

A mixture of 100 mg (17beta)-17-(pyrimidin-5-yl)estra-1,3,5(10)-triene-3-carboxylic acid (intermediate 1-4) and 88 mg tert-butyl N-methyl-beta-alaninate in 3 ml THF and 1 ml DMF was treated with 42 mg 1-hydroxybenzotriazole hydrate, 106 mg 1-ethyl-3-(3-dimethylaminopropyl)carbodiimide hydrochloride and 0.12 ml triethylamine and was stirred for 18 h. Water was added and the mixture was extracted three times by ethyl acetate. The organic layer was evaporated leading to 173 mg tert-butyl N-methyl-N-{[(17beta)-17-(pyrimidin-5-yl)estra-1,3,5(10)-trien-3-yl]carbonyl}-beta-alaninate (crude product).

### EXPERIMENTAL SECTION -EXAMPLES

### Example 1

### N-{[(17beta)-17-(5-fluoropyridin-3-yl)estra-1,3,5(10)-trien-3-yl]carbonyl}-N-methyl-beta-alanine

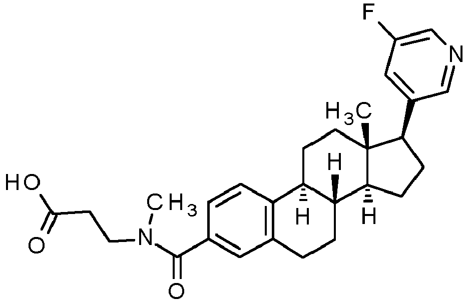

To a solution of 850 mg tert-butyl N-{[(17beta)-17-(5-fluoropyridin-3-yl)estra-1,3,5(10)-trien-3-yl]carbonyl}-N-methyl-beta-alaninate (intermediate 2-1) in 15 ml dichloromethane 1.3 ml trifluoroacetic acid was added and the mixture was stirred at 40°C over night, then poured on ice-cold water and stirred for 30 min. After extraction with dichloromethane, the combined organic layers were concentrated and the residue was stirred in diethylether, filtered, washed three times with diethylether and dried affording 710 mg N-{[(17beta)-17-(5-fluoropyridin-3-yl)estra-1,3,5(10)-trien-3-yl]carbonyl}-N-methyl-beta-alanine.

¹H-NMR (300MHz, DMSO-d₆): δ [ppm]= 0.44 (s, 3H), 1.24 - 1.56 (m, 7H), 1.76 - 2.01 (m, 3H), 2.08 - 2.36 (m), 2.77 - 2.92 (m, 6H), 3.57 (br s), 7.00 - 7.09 (m, 2H), 7.27 (d, 1H), 7.58 - 7.68 (m, 1H), 8.31 (s, 1H), 8.38 (d, 1H), 12.3 (br s, 1H).

### Example 2

### N-[[(17beta)-17-(5-fluoropyridin-3-yl)estra-1,3,5(10)-trien-3-yl]carbonyl}glycine

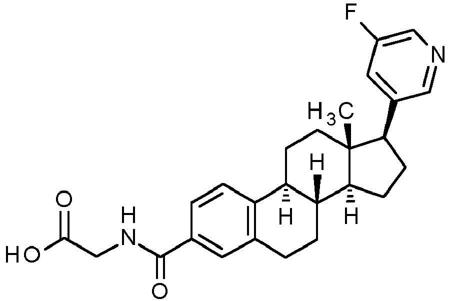

To a mixture of 100 mg (17beta)-17-(5-fluoropyridin-3-yl)estra-1,3,5(10)-triene-3-carboxylic acid (Intermediate 1-1) in 3.0 ml THF and 1.0 ml DMF 66 mg methyl glycinate hydrochloride (1:1), 40 mg 1-hydroxybenzotriazole hydrate, 101 mg 1-ethyl-3-(3-dimethylaminopropyl)carbodiimide hydrochloride and 0.11 ml triethylamine were added and the mixture was stirred at room temperature over night. 1.3 ml 2M aqeous sodium hydroxide solution and 0.50 ml methanol were added and the mixture was stirred for 3 h at 40°C. Water was added and aqeous citric acid solution (10%) was added leading to a pH value in the range between 3 and 4. Extraction by ethyl acetate, evaporation and purification by HPLC afforded 22 mg of the title compound.

¹H-NMR (300MHz, DMSO-d₆): δ [ppm]= 0.44 (s, 3H), 1.20 - 1.57 (m, 7H), 1.78 - 1.99 (m, 3H), 2.08 - 2.41 (m, 3H), 2.77 - 2.92 (m, 3H), 3.85 (d, 2H), 7.33 (d, 1H), 7.51 - 7.69 (m, 3H), 8.31 (s, 1H), 8.39 (d, 1H), 8.66 (t, 1H), 12.54 (br s).

### Example 3

### N-{[(17beta)-17-(5-fluoropyridin-3-yl)estra-1,3,5(10)-trien-3-yl]carbonyl}-beta-alanine

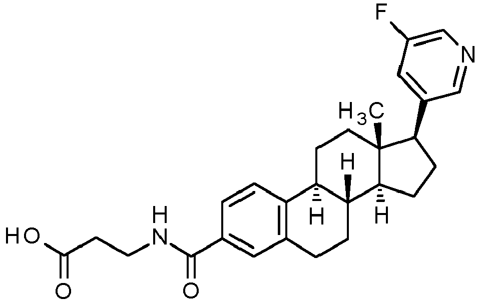

To a mixture of 100 mg (17beta)-17-(5-fluoropyridin-3-yl)estra-1,3,5(10)-triene-3-carboxylic acid (Intermediate 1-1) in 3.0 ml THF and 1.0 ml DMF 81 mg ethyl beta-alaninate hydrochloride (1:1), 40 mg 1-hydroxybenzotriazole hydrate, 101 mg 1-ethyl-3-(3-dimethylaminopropyl)carbodiimide hydrochloride and 0.11 ml triethylamine were added and the mixture was stirred at room temperature over night. 1.3 ml 2M aqeous sodium hydroxide solution and 0.50 ml methanol were added and the mixture was stirred for 3 h at 40°C. Water was added and aqeous citric acid solution (10%) was added leading to a pH value in the range between 3 and 4. Extraction by ethyl acetate, evaporation and purification by HPLC afforded 74 mg of the title compound.

¹H-NMR (400MHz, DMSO-d₆): δ [ppm]= 0.44 (s, 3H), 1.21 - 1.56 (m, 7H), 1.78 - 1.98 (m, 3H), 2.09 - 2.22 (m, 1H), 2.22 - 2.36 (m, 2H), 2.41 - 2.44 (m), 2.79 - 2.90 (m, 3H), 3.36 - 3.46 (m), 7.29 (d, 1H), 7.49 - 7.56 (m, 2H), 7.58 - 7.65 (m, 1H), 8.28 - 8.41 (m, 3H).

### Example 4

### 4-({[(17beta)-17-(5-fluoropyridin-3-yl)estra-1,3,5(10)-trien-3-yl]carbonyl}amino)butanoic acid

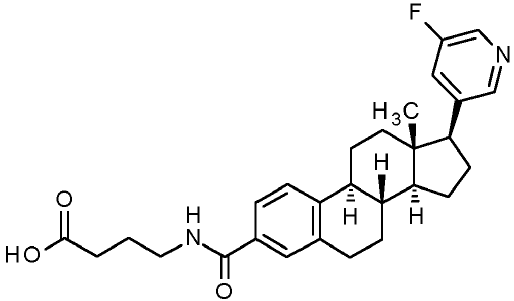

To a mixture of 100 mg (17beta)-17-(5-fluoropyridin-3-yl)estra-1,3,5(10)-triene-3-carboxylic acid (Intermediate 1-1) in 3.0 ml THF and 1.0 ml DMF 81 mg methyl 4-aminobutanoate hydrochloride (1:1), 40 mg 1-hydroxybenzotriazole hydrate, 101 mg 1-ethyl-3-(3-dimethylaminopropyl)carbodiimide hydrochloride and 0.11 ml triethylamine were added and the mixture was stirred at room temperature over night. 1.3 ml 2M aqeous sodium hydroxide solution and 0.50 ml methanol were added and the mixture was stirred for 3 h at 40°C. Water was added and aqeous citric acid solution (10%) was added leading to a pH value in the range between 3 and 4. Extraction by ethyl acetate, evaporation and purification by HPLC afforded 74 mg of the title compound.

¹H-NMR (400MHz, DMSO-d₆): δ [ppm]= 0.44 (s, 3H), 1.22 - 1.55 (m, 7H), 1.70 (quin, 2H), 1.80 - 1.98 (m, 3H), 2.10 - 2.36 (m, 5H), 2.79 - 2.90 (m, 3H), 3.16 - 3.24 (m), 7.29 (d, 1H), 7.54 (d, 1H), 7.52 (s, 1H), 7.62 (br d, 1H), 8.28 - 8.34 (m, 2H), 8.38 (d, 1H), 12.03 (br s, 1H).

### Example 5

### 3-([[(17beta)-17-(5-fluoropyridin-3-yl)estra-1,3,5(10)-trien-3-yl]carbonyl}amino)-2,2-dimethylpropanoic acid

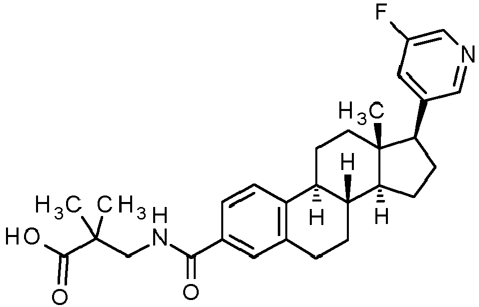

To a mixture of 100 mg (17beta)-17-(5-fluoropyridin-3-yl)estra-1,3,5(10)-triene-3-carboxylic acid (Intermediate 1-1) in 3.0 ml THF and 1.0 ml DMF 81 mg methyl 3-amino-2,2-dimethylpropanoate hydrochloride (1:1), 40 mg 1-hydroxybenzotriazole hydrate, 101 mg 1-ethyl-3-(3-dimethylaminopropyl)carbodiimide hydrochloride and 0.11 ml triethylamine were added and the mixture was stirred at room temperature for 3.5 h. 0.66 ml 2M aqueous sodium hydroxide solution and 0.10 ml methanol were added and the mixture was stirred for 3 days at room temperature. Then 0.66 ml 2M aqueous sodium hydroxide solution were added and the mixture was stirred for 4 h. Water was added and aqeous citric acid solution (10%) was added leading to a pH value in the range between 3 and 4. Extraction by ethyl acetate, evaporation and purification by HPLC afforded 84 mg of the title compound.

¹H-NMR (300MHz, DMSO-d₆): δ [ppm]= 0.44 (s, 3H), 1.05 (s, 6H), 1.19 - 1.56 (m, 7H), 1.74 - 2.00 (m, 3H), 2.08 - 2.38 (m, 3H), 2.77 - 2.91 (m, 3H), 3.33 - 3.41 (m, 2H), 7.30 (d, 1H), 7.48 - 7.55 (m, 2H), 7.57 - 7.68 (m, 1H), 8.11 (t, 1H), 8.28 - 8.33 (m, 1H), 8.38 (d, 1H), 12.22 (br s, 1H).

### Example 6

### N-{[(17beta)-17-(5-methoxypyridin-3-yl)estra-1,3,5(10)-trien-3-yl]carbonyl}-N-methyl-beta-alanine

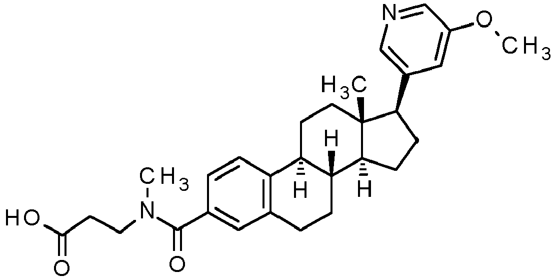

A mixture of 136 mg tert-butyl N-{[(17beta)-17-(5-methoxypyridin-3-yl)estra-1,3,5(10)-trien-3-yl]carbonyl}-N-methyl-beta-alaninate (intermediate 2-3) in 3.0 ml dichloromethane and 1.0 ml trifluoroacetic acid was stirred at room temperature over night. Evaporation of solvents and purification by HPLC afforded 55 mg N-{[(17beta)-17-(5-methoxypyridin-3-yl)estra-1,3,5(10)-trien-3-yl]carbonyl}-N-methyl-beta-alanine.

### Example 7

### N-methyl-N-{[(17beta)-17-[5-(trifluoromethyl)pyridin-3-yl]estra-1,3,5(10)-trien-3-yl]carbonyl}-beta-alanine

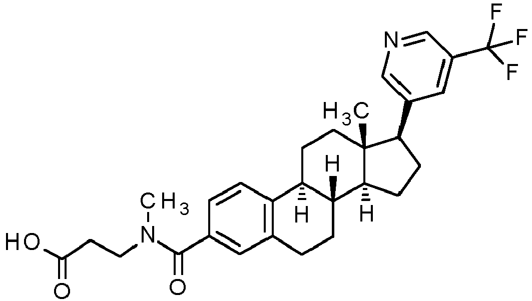

A mixture of 141 mg t tert-butyl N-methyl-N-{[(17beta)-17-[5-(trifluoromethyl)pyridin-3-yl]estra-1,3,5(10)-trien-3-yl]carbonyl}-beta-alaninate (intermediate 2-2) in 3.0 ml dichloromethane and 0.5 ml trifluoroacetic acid was stirred at room temperature for three days. Evaporation of solvents and purification by HPLC afforded 68 mg N-methyl-N-{[(17beta)-17-[5-(trifluoromethyl)pyridin-3-yl]estra-1,3,5(10)-trien-3-yl]carbonyl}-beta-alanine.

¹H-NMR (400MHz, DMSO-d₆): δ [ppm]= 0.44 (s, 3H), 1.18 - 1.60 (m, 7H), 1.78 - 2.02 (m, 3H), 2.18 - 2.36 (m, 3H), 2.48 - 2.53 (m), 2.76 - 2.96 (m, 6H), 3.41 (br s, 1H), 3.56 (br s, 1H), 7.03 (s, 1H), 7.06 (d, 1H), 7.27 (d, 1H), 8.00 (s, 1H), 8.75 (d, 1H), 8.79 (d, 1H), 12.26 (br s, 1H).

### Example 8

### N-methyl-N-[[(17beta)-17-[5-(trifluoromethyl)pyridin-3-yl]estra-1,3,5(10)-trien-3-yl]carbonyl}glycine

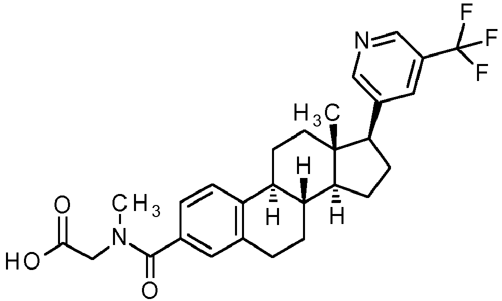

To a mixture of 100 mg (17beta)-17-[5-(trifluoromethyl)pyridin-3-yl]estra-1,3,5(10)-triene-3-carboxylic acid (intermediate 1-2) in 3.0 ml THF and 0.5 ml DMF 65 mg methyl N-methylglycinate hydrochloride (1:1), 36 mg 1-hydroxybenzotriazole hydrate, 89 mg 1-ethyl-3-(3-dimethylaminopropyl)carbodiimide hydrochloride and 0.10 ml triethylamine were added and the mixture was stirred at room temperature over night. 0.58 ml 2M aqeous sodium hydroxide solution and 0.50 ml methanol were added and the mixture was stirred for 4 h at 50°C. Water was added and aqeous citric acid solution (10%) was added leading to a pH value of 4. Extraction by ethyl acetate, evaporation and purification by HPLC afforded 70 mg of the title compound.

¹H-NMR (300MHz, DMSO-d₆): δ [ppm]= 0.44 (s, 3H), 1.19 - 1.63 (m, 7H), 1.79 - 2.02 (m, 3H), 2.17 - 2.37 (m), 2.73 - 2.98 (m, 6H, contains singulett at 2.91 ppm), 3.90 (br s), 4.07 (s), 6.94 - 7.16 (m, 2H), 7.22 - 7.35 (m, 1H), 8.00 (s, 1H), 8.75 (s, 1H), 8.79 (s, 1H), 12.75 (br s, 1H).

### Example 9

### N-{[(17beta)-17-[5-(trifluoromethyl)pyridin-3-yl]estra-1,3,5(10)-trien-3-yl]carbonyl}-beta-alanine

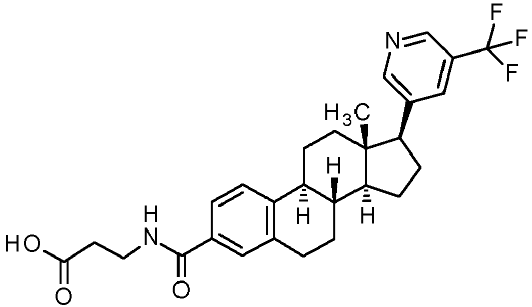

To a mixture of 100 mg (17beta)-17-[5-(trifluoromethyl)pyridin-3-yl]estra-1,3,5(10)-triene-3-carboxylic acid (intermediate 1-2) in 3.0 ml THF and 0.5 ml DMF 72 mg ethyl beta-alaninate hydrochloride (1:1), 36 mg 1-hydroxybenzotriazole hydrate, 89 mg 1-ethyl-3-(3-dimethylaminopropyl)carbodiimide hydrochloride and 0.10 ml triethylamine were added and the mixture was stirred at room temperature over night. 0.58 ml 2M aqeous sodium hydroxide solution and 0.50 ml methanol were added and the mixture was stirred for 3 h at 40°C. Water and aqeous citric acid solution (10%) were added leading to a pH value of 4. Extraction by ethyl acetate, evaporation and purification by HPLC afforded 60 mg of the title compound.

¹H-NMR (300MHz, DMSO-d₆): δ [ppm]= 0.44 (s, 3H), 1.19 - 1.61 (m, 7H), 1.78 - 2.04 (m, 3H), 2.17 - 2.38 (m, 3H), 2.38 - 2.43 (m), 2.79 - 2.97 (m, 3H), 3.34 - 3.45 (m, 2H), 7.29 (d, 1H), 7.48 - 7.55 (m, 2H), 8.00 (s, 1H), 8.34 (t, 1H), 8.74 (br s, 1H), 8.79 (s, 1H), 12.2 (br s).

### Example 10

### N-methyl-N-{[(17beta)-17-(pyrimidin-5-yl)estra-1,3,5(10)-trien-3-yl]carbonyl}-beta-alanine

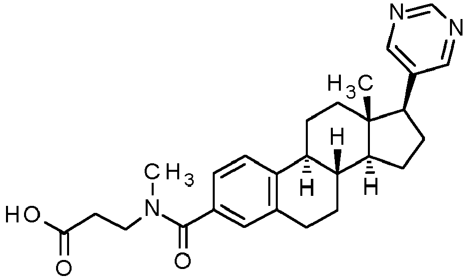

A mixture of 139 mg tert-butyl N-methyl-N-{[(17beta)-17-(pyrimidin-5-yl)estra-l,3,5(10)-trien-3-yl]carbonyl}-beta-alaninate (intermediate 2-4) in 3.0 ml dichloromethane and 1.0 ml trifluoroacetic acid was stirred at room temperature over night. Evaporation of solvents and purification by HPLC afforded 53 mg N-methyl-N-{[(17beta)-17-(pyrimidin-5-yl)estra-l,3,5(10)-trien-3-yl]carbonyl}-beta-alanine.

¹H-NMR (400MHz, DMSO-d₆): δ [ppm]= 0.46 (s, 3H), 1.22 - 1.55 (m, 7H), 1.80 - 1.99 (m, 3H), 2.15 - 2.34 (m, 3H), 2.49 - 2.55 (m), 2.72 - 2.91 (m, 6H), 3.40 (br s, 1H), 3.56 (br s, 1H), 7.00 - 7.09 (m, 2H), 7.27 (d, 1H), 8.69 (s, 2H), 9.01 (s, 1H), 12.29 (br s, 1H).

### Example 11

### N-{[(15beta,17beta)-17-(5-fluoropyridin-3-yl)-15-hydroxyestra-1,3,5(10)-trien-3-yl]carbonyl}-N-methyl-beta-alanine

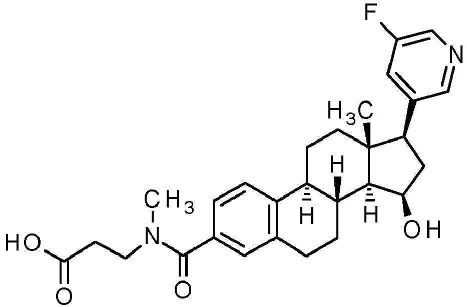

A 100 ml Erlenmeyer flask which contained 20 ml of an aqueous nutrient solution comprising 3% of glucose monohydrate, 1% maize steeping liquor, 0.2% sodium nitrate, 0.1% potassium dihydrogen phosphate, 0.2% dipotassium hydrogen phosphate, 0.05% potassium chloride, 0.05% magnesium sulphate heptahydrate and 0.002% iron(II) sulphate heptahydrate (adjusted to pH 6.0) which had been sterilized in an autoclave at 121°C for 20 minutes was inoculated with 0.2 ml of a DMSO/ice culture of the strain Mucor plumbeus (CBS No. 29563) and shaken at 27°C on a rotary shaker at 165 revolutions per minute for 48 hours. A 500 ml Erlenmeyer flask which had been charged with 100 ml of sterile medium of the same final composition as described for the preculture was inoculated with 8 ml of this preculture. This flask was shaken at 27°C on a rotary shaker at 165 rotations per minute for 72 hours. Two 21 Erlenmeyer flasks each containing 11 of a sterile nutrient solution comprising 3% glucose monohydrate, 1% ammonium chloride, 0.2% sodium nitrate, 0.1% potassium dihydrogen phosphate, 0.2% dipotassium hydrogen phosphate, 0.05% potassium chloride, 0.05% magnesium sulphate heptahydrate and 0.002% iron(II) sulphate heptahydrate were inoculated with in each case 50 ml of this preculture. After a growth phase of 6 hours on a rotary shaker at 165 revolutions per minute at a temperature of 27°C, a solution of 100 mg of N-{[(17beta)-17-(5-fluoropyridin-3-yl)estra-1,3,5(10)-trien-3-yl]carbonyl}-N-methyl-beta-alanine (Example 1) in 10 ml of DMF was divided into the two flasks. The flasks were shaken for a further 47 hours and then worked up. The two culture broths were combined and extracted with 11 of isobutyl methyl ketone at 40 revolutions per minute in a 51 glass extracting vessel for 19 hours. The organic phase was dried over sodium sulphate and concentrated to dryness. This gave 334 mg of a crude product as brown oil. The crude product was absorbed on diatomaceous earth and chromatographed: instrument: Biotage Isolera, 10 g SNAP column, solvent: gradient from 2 to 20% methanol in ethyl acetate (1% of glacial acetic acid added) to yield 101 mg of an enriched product. Subsequent HPLC purification gave 65 mg (62% yield) of the target compound.

1H-NMR (600MHz, METHANOL-d4): δ [ppm]= 0.79 (s, 6H), 1.41 (dd, 2H), 1.43 - 1.60 (m, 6H), 1.62 - 1.69 (m, 2H), 1.78 - 1.89 (m, 2H), 2.13 - 2.21 (m, 2H), 2.27 - 2.35 (m, 2H), 2.35 - 2.46 (m, 3H), 2.55 (br. s., 1H), 2.60 (dt, 1H), 2.69 (br. s., 1H), 2.81 - 2.88 (m, 1H), 2.89 - 3.07 (m, 5H), 3.56 - 3.83 (m, 2H), 4.49 - 4.56 (m, 1H), 7.09 - 7.18 (m, 2H), 7.37 (d, 1H), 7.58 (dt, 1H), 8.27 - 8.36 (m, 2H).

### Analytics

| | | | |
|---|---|---|---|
| *System:* | Waters Aqcuity UPLC-MS: Binary Solvent Manager, Sample Manager/Organizer, Column Manager, PDA, ELSD, SQD 3100 | | |
| *Säule:* | Aqcuity BEH C18 1.7 50x2.1mm | | |
| *solvent:* | A = H₂O + 0.1% HCOOH | | |
| | B = acetonitrile | | |
| *gradient:* | 0-1.6 min 1-99% B, 1.6-2.0 min 99% B | | |
| *flow:* | 0.8 ml/min | | |
| *temperature:* | 60°C | | |
| *solution:* | 1.0 mg/ml EtOH/MeOH 2:1 | | |
| *injection:* | 2.0 µl | | |
| *detection:* | DAD TAC, scan range 210-400 nm | | |
| | MS ESI+, ESI-, scan range 160-1000 m/z | | |
| | ELSD | | |

| Peak | Rt in min | Purity in % | Mass m/z |
|---|---|---|---|
| 5 | 1.02 | 90 | 480 |

### Preparation

| | | | | |
|---|---|---|---|---|
| *system:* | Waters Autopurificationsystem: Pump 254, Sample Manager 2767, CFO, DAD 2996, ELSD 2424, SQD 3100 | | | |
| *column:* | XBrigde C18 5µm 100x30 mm | | | |
| *solvent:* | A = H₂O + 0.1% HCOOH | | | |
| | B = acetonitrile | | | |
| *gradient:* | 0-8 min 15-50% B | | | |
| *flow:* | 50 ml/min | | | |
| *temperature:* | RT | | | |
| *solution:* | 100 mg / 2 ml DMF/MeOH 1+1 | | | |
| *injection:* | 3 x 0.667 ml | | | |
| *detection:* | DAD scan range 210-400 nm | | | |
| | MS ESI+, ESI-, scan range 160-1000 m/z | | | |

| fractions | Rt in min | Purity in % | Amount in mg | Peak Assignment |
|---|---|---|---|---|
| 11 | 5.91-6.40 | 98 | 65 | 5 (1.02 min) |
| *work-up:* | The fractions were concentrated, treated with tBuOH and freeze-dried | | | |

### Example 12

### N-{[(15alpha,17beta)-17-(5-fluoropyridin-3-yl)-15-hydroxyestra-1,3,5(10)-trien-3-yl]carbonyl}-N-methyl-beta-alanine

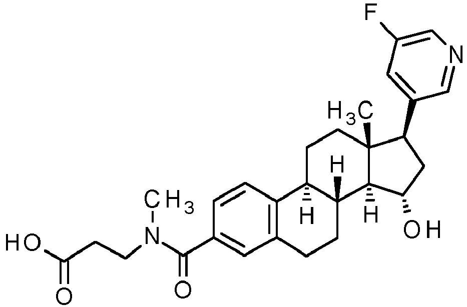

A 100 ml Erlenmeyer flask which contained 20 ml of an aqueous nutrient solution comprising, 1% maize steeping liquor, 1.25% soybean meal (adjusted to pH 6.2) which had been sterilized in an autoclave at 121 °C for 20 minutes was inoculated with 0.2 ml of a DMSO/ice culture of the strain Calonectria decora (ATCC No. 14767) and shaken at 20°C on a rotary shaker at 165 revolutions per minute for 72 hours. Two 500 ml Erlenmeyer flask which had been charged with 100 ml of sterile medium of the same final composition as described for the preculture was inoculated with 8 ml of this preculture. These flasks were shaken at 20°C on a rotary shaker at 165 rotations per minute for 72 hours. Three 21 Erlenmeyer flasks each containing 11 of a sterile nutrient solution comprising 3% glucose monohydrate, 1% ammonium chloride, 0.2% sodium nitrate, 0.1% potassium dihydrogen phosphate, 0.2% dipotassium hydrogen phosphate, 0.05% potassium chloride, 0.05% magnesium sulphate heptahydrate and 0.002% iron(II) sulphate heptahydrate were inoculated with in each case 50 ml of this preculture. After a growth phase of 6 hours on a rotary shaker at 165 revolutions per minute at a temperature of 27°C, a solution of 75 mg of N-{[(17beta)-17-(5-fluoropyridin-3-yl)estra-1,3,5(10)-trien-3-yl]carbonyl}-N-methyl-beta-alanine (Example 1) in 7.5 ml of DMF was divided into the three flasks. The flasks were shaken for a further 44 hours. The three culture broths were then combined and extracted with 1.51 of isobutyl methyl ketone at 40 revolutions per minute in a 51 glass extracting vessel for 19 hours. The organic phase was dried over sodium sulphate and concentrated to dryness. This gave 276 mg of a crude product as brown oil. The crude product was absorbed on diatomaceous earth and chromatographed: instrument: Biotage Isolera, 10 g SNAP column, solvent: gradient from 2 to 20% methanol in ethyl acetate (1% of glacial acetic acid added) to yield 60 mg of an enriched product fraction. Subsequent HPLC purification gave 28 mg (34% yield) of the target compound.

¹H-NMR (600MHz, METHANOL-d4): δ [ppm]= 0.59 (s, 3H), 1.37 - 1.48 (m, 1H), 1.52 - 1.62 (m, 3H), 1.63 - 1.72 (m, 2H), 1.89 (ddd, 1H), 2.35 - 2.45 (m, 3H), 2.51 - 2.72 (m, 2H), 2.79(ddd, 1H), 2.93 (dd, 2H), 2.99 - 3.09 (m, 3H), 3.15 - 3.20 (m, 1H), 3.56 - 3.82 (m, 2H), 4.26 (td, 1H), 7.11 (br. s., 1H), 7.14 (d, 1H), 7.36 (d, 1H), 7.59 (dt, 1H), 8.31 (d, 2H).

### Analytics

| | | | |
|---|---|---|---|
| *system:* | Waters Aqcuity UPLC-MS: Binary Solvent Manager, Sample Manager/Organizer, Column Manager, PDA, ELSD, SQD 3100 | | |
| *colum:* | Aqcuity BEH C18 1.7 50x2.1mm | | |
| *solvent:* | A = H₂O + 0.1% HCOOH | | |
| | B = acetonitrile | | |
| *gradient:* | 0-1.6 min 1-99% B, 1.6-2.0 min 99% B | | |
| *flow:* | 0.8 ml/min | | |
| *temperature:* | 60°C | | |
| *solution:* | 1.0 mg/ml EtOH/MeOH 2:1 | | |
| *injection:* | 2.0 µl | | |
| *detection:* | DAD TAC, scan range 210-400 nm | | |
| | MS ESI+, ESI-, scan range 160-1000 m/z | | |
| | ELSD | | |

| Peak | Rt in min | purity in % | mass m/z |
|---|---|---|---|
| 4 | 0.96 | 70 | 480 |

### Preparation

| | | | | |
|---|---|---|---|---|
| *system:* | Waters Autopurificationsystem: Pump 254, Sample Manager 2767, CFO, DAD 2996, ELSD 2424, SQD 3100 | | | |
| *column:* | XBrigde C18 5µm 100x30 mm | | | |
| *solvent:* | A = H₂O + 0.1% HCOOH | | | |
| | B = Acetonitril | | | |
| *gradient:* | 0-8 min 15-50% B | | | |
| *flow:* | 50 ml/min | | | |
| *temperature:* | RT | | | |
| *solution:* | 59 mg / 2 ml DMF/MeOH 1+1 | | | |
| *injection:* | 2 x 1 ml | | | |
| *detection:* | DAD scan range 210-400 nm | | | |
| | MS ESI+, ESI-, scan range 160-1000 m/z | | | |

| fractions | Rt in min | purity in % | amount in mg | peak assignment |
|---|---|---|---|---|
| 11 | 5.21-5.76 | >99 | 28 | 4 (0.96 min) |
| *work-up:* | The fractions were concentrated, treated with tBuOH and freeze-dried | | | |

### EXPERIMENTAL SECTION -BIOLOGICAL ASSAYS

### AKR1C3-inhibitory action

The AKR1C3-inhibitory action of the substances of this invention was measured in the AKR1C3 assay described in the paragraphs which follow.

Essentially, the enzyme activity is measured by quantifying the coumberol formed from coumberone (Halim, M., Yee, D.J., and Sames, D., J. AM. CHEM. SOC. 130, 14123-14128 (2008) and Yee, D.J., Balsanek, V., Bauman, D.R., Penning, T.M., and Sames, D., Proc. Natl. Acad. Sci. USA 103, 13304 - 13309 (2006)). In this assay, the increase in the highly fluorescent coumberol can be determined by NADPH (nicotinamide adenine dinucleotide phosphate)-dependent reduction of the nonfluorescent coumberone by AKR1C3.

The enzyme used was recombinant human AKR1C3 (aldo-keto reductase family 1 member C3) (GenBank Accession No. NM_003739). This was expressed as the GST (glutathione S-transferase) fusion protein in *E. coli* and purified by means of glutathione-Sepharose affinity chromatography. The GST was removed by thrombin digestion with subsequent size exclusion chromatography (Dufort, I., Rheault, P., Huang, XF., Soucy, P., and Luu-The, V., Endocrinology 140, 568-574 (1999)).

For the assay, 50 nl of a 100-fold concentrated solution of the test substance in DMSO were pipetted into a black low-volume 384-well microtitre plate (Greiner Bio-One, Frickenhausen, Germany), 2.0 µl of a solution of AKR1C3 in assay buffer [50 mM potassium phosphate buffer pH 7, 1 mM DTT, 0.0022% (w/v) Pluronic F-127, 0.01% BSA (w/v) and protease inhibitor cocktail (complete, EDTA-free Protease Inhibitor Cocktail from Roche)] were added and the mixture was incubated for 15 min, in order to enable preliminary binding of the substances to the enzyme prior to the enzyme reaction. Then the enzyme reaction was started by adding 3 µl of a solution of NADPH (16.7 µM → final concentration in assay volume 5 µl is 10 µM) and coumberone (0.5 µM → final concentration in assay volume 5 µl is 0.3 µM) in assay buffer and the resulting mixture was incubated at 22°C for the reaction time of 90 min. The concentration of the AKR1C3 was matched to the respective activity of the enzyme preparation and set such that the assay worked within the linear range. Typical concentrations were in the region of 1 nM. The reaction was stopped by adding 5 µl of a stop solution consisting of the inhibitor EM-1404 [F. Labrie et al. US Patent 6,541,463, 2003] (2 µM → final concentration in assay volume 5 µl is 1 µM). Subsequently, the fluorescence of coumberol was measured at 520 nm (excitation at 380 nm) with a suitable measuring instrument (Pherastar from BMG Labtechnologies). The intensity of the fluorescence was used as a measure for the amount of coumberol formed and hence for the enzyme activity of AKR1C3. The data were normalized (enzyme reaction without inhibitor = 0% inhibition; all other assay components but no enzyme = 100% inhibition). Typically, the test substances were tested on the same microtitre plate at 11 different concentrations in the range from 20 µM to 96.8 pM (20 µM, 5.9 µM, 1.7 µM, 0.5 µM, 0.15 µM, 44 nM, 12.9 nM, 3.8 nM, 1.1 nM, 0.3 nM and 96.8 pM; the dilution series were prepared before the assay at the level of the 100-fold concentrated solution by serial 1:3 dilutions with 100% DMSO) in twin values for each concentration, and IC₅₀ values were calculated by a 4-parameter fit.

As described, the pharmacological substances claimed were tested for their inhibitory effect on the AKR1C3 enzyme (see Table 3). The compounds showed strong inhibition of AKRIC3 *in vitro* (IC₅₀ values ≤300 nM).

**Table 3: Inhibition of AKR1C3 by the inventive compounds (for some of the compounds, the values for two experimental determinations are reported)**

| Example compound | AKR1C3 enzyme inhibition IC₅₀ [nmol/l] |
|---|---|
| 1 | 19.2 / 23.7 |
| 2 | 26.4 / 28.9 |
| 3 | 5.47 / 9.35 |
| 4 | 7.79 / 6.76 |
| 5 | 13.8 / 13.1 |
| 6 | 67.6 / 82.1 |
| 7 | 35.0 / 44.1 |
| 8 | 48.5 / 76.2 |
| 9 | 16.9 / 26.0 |
| 10 | 271 / 282 |
| 11 | 22.4 |
| 12 | 188 |

## Claims

1. A compound of general formula (I): 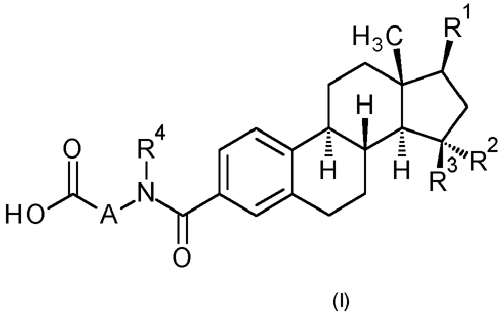 in which
R¹ repesents a pyridine, pyrimidine, pyridazine or pyrazine moiety which is substituted with R⁵;
R² represents hydrogen or hydroxyl;
R³ represents hydrogen or hydroxyl,
wherein only one of R² and R³ represents hydroxyl;
R⁴ represents hydrogen or methyl;
R⁵ represents hydrogen, fluoro, chloro, cyano, C₁-C₄-alkyl, trifluoromethyl or methoxy;
A represents -(CR^{a}R^{b})ₙ-
wherein R^{a} and R^{b}, independently from each other, represent hydrogen or methyl;
n represents 1, 2, 3 or 4;
and the hydrates, salts, solvates and solvates of the salts thereof.

2. The compound according to claim 1, wherein:
R¹ represents pyrimidin-5-yl, pyridazin-4-yl, 6-methylpyridazin-4-yl or a group 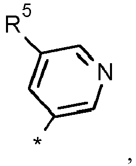
wherein * indicates the point of attachment of said group with the rest of the molecule;
R² represents hydrogen or hydroxyl;
R³ represents hydrogen or hydroxyl;
wherein only one of R² and R³ represents hydroxyl;
R⁴ represents hydrogen or methyl;
R⁵ represents hydrogen, fluoro, chloro, methyl, trifluoromethyl or methoxy;
A represents -(CR^{a}R^{b})ₙ-wherein R^{a} and R^{b}, independently from each other, represent hydrogen or methyl;
n represents 1, 2, or 3;
and the hydrates, salts, solvates and solvates of the salts thereof.

3. The compound according to claim 1 or 2, wherein:
R¹ represents a group selected from: 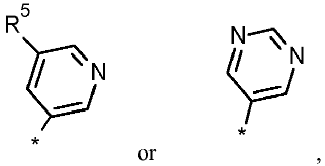 wherein * indicates the point of attachment of said group with the rest of the molecule;
R² represents hydrogen or hydroxyl;
R³ represents hydrogen or hydroxyl;
wherein only one of R² and R³ represents hydroxyl;
R⁴ represents hydrogen or methyl;
R⁵ represents fluoro, trifluoromethyl or methoxy;
A represents -CH₂-, -CH₂-CH₂-, -CH₂-CH₂-CH₂- or -C(CH₃)₂-CH₂-;
and the hydrates, salts, solvates and solvates of the salts thereof.

4. The compound according to claim 1, 2 or 3 which is selected from the group consisting of:
- N-{[(17beta)-17-(5-fluoropyridin-3-yl)estra-1,3,5(10)-trien-3-yl]carbonyl}-N-methyl-beta-alanine
- N-{[(17beta)-17-(5-fluoropyridin-3-yl)estra-1,3,5(10)-trien-3-yl]carbonyl}glycine
- N-{[(17beta)-17-(5-fluoropyridin-3-yl)estra-1,3,5(10)-trien-3-yl]carbonyl}-beta-alanine
- 4-({[(17beta)-17-(5-fluoropyridin-3-yl)estra-1,3,5(10)-trien-3-yl]carbonyl}amino)butanoic acid
- 3-({[(17beta)-17-(5-fluoropyridin-3-yl)estra-1,3,5(10)-trien-3-yl]carbonyl}amino)-2,2-dimethylpropanoic acid
- N-{[(17beta)-17-(5-methoxypyridin-3-yl)estra-1,3,5(10)-trien-3-yl]carbonyl}-N-methyl-beta-alanine
- N-methyl-N-{[(17beta)-17-[5-(trifluoromethyl)pyridin-3-yl]estra-1,3,5(10)-trien-3-yl]carbonyl}-beta-alanine
- N-methyl-N-{[(17beta)-17-[5-(trifluoromethyl)pyridin-3-yl]estra-1,3,5(10)-trien-3-yl]carbonyl} glycine
- N-{[(17beta)-17-[5-(trifluoromethyl)pyridin-3-yl]estra-1,3,5(10)-trien-3-yl]carbonyl}-beta-alanine
- N-methyl-N-{[(17beta)-17-(pyrimidin-5-yl)estra-1,3,5(10)-trien-3-yl]carbonyl}-beta-alanine
- N-{[(15beta,17beta)-17-(5-fluoropyridin-3-yl)-15-hydroxyestra-1,3,5(10)-trien-3-yl]carbonyl}-N-methyl-beta-alanine
- N-{[(15alpha,17beta)-17-(5-fluoropyridin-3-yl)-15-hydroxyestra-1,3,5(10)-trien-3-yl]carbonyl}-N-methyl-beta-alanine
and the hydrates, salts, solvates and solvates of the salts thereof.

5. A compound of general formula (I) according to any one of claims 1 to 4 for use in the treatment or prophylaxis of a disease.

6. A pharmaceutical composition comprising a compound of general formula (I) according to any one of claims 1 to 4 and one or more pharmaceutically acceptable excipients.

7. A pharmaceutical combination comprising (a) one or more first active ingredients, in particular compounds of general formula (I) according to any one of claims 1 to 4, and (b) one or more further active ingredient.

8. A pharmaceutical combination according to claim 7, wherein said further active ingredient is selected from the group of anti-androgens, CYP17A1 inhibitors, 5 alpha reductase inhibitors, GNRHa and GNRH antagoists, or LHRH agonists for example Flutamide, Bicalutamide, Nilutamide, Enzaluatmide, ODM-201, Abiraterone and abiraterone metabolites, finasteride dutasteride, Leuprolide, Goserelin, Triptorelin, Histrelin, or Degarelix.

9. Use of a compound of general formula (I) according to any one of claims 1 to 4 or a combination according to claim 7 or 8 for the treatment or prophylaxis of a disease.

10. Use of a compound of general formula (I) according to any one of claims 1 to 4 or a combination according to claim 7 or 8 for the preparation of a medicament for the treatment or prophylaxis of a disease.

11. Use according to claim 9 or 10, wherein the disease is a gynecological disorder.

12. Use according to claim 11, wherein the gynecological disease is endometriosis.

13. Use according to claim 9 or 10, wherein the disease is a hyperproliferative disorder, a metabolic disorder or an inflammatory disorder such as endometriosis-related or polycystic ovary syndrome-related gynecological disorder, condition or disease, atopic dermatitis, anthracycline resistant cancer or prostate cancer including castration-resistant prostate cancer.
